(19) Europäisches Patentamt
European Patent Office
Office européen des brevets

(11) EP 0 665 832 B1

# (12) EUROPÄISCHE PATENTSCHRIFT

(45) Veröffentlichungstag und Bekanntmachung des Hinweises auf die Patenterteilung:
**16.04.1997 Patentblatt 1997/16**

(21) Anmeldenummer: **93923467.0**

(22) Anmeldetag: **14.10.1993**

(51) Int Cl.6: **C07C 319/20**, C07C 323/58

(86) Internationale Anmeldenummer:
**PCT/EP93/02838**

(87) Internationale Veröffentlichungsnummer:
**WO 94/08957 (28.04.1994 Gazette 1994/10)**

## (54) KONTINUIERLICH DURCHFÜHRBARES VERFAHREN ZUR HERSTELLUNG VON METHIONIN ODER METHIONINDERIVATEN

METHOD FOR THE CONTINUOUS PREPARATION OF METHIONINE OR METHIONINE DERIVATIVES

PROCEDE EXECUTABLE EN CONTINU POUR PREPARER DE LA METHIONINE OU DES DERIVES DE LA METHIONINE

(84) Benannte Vertragsstaaten:
**BE CH DE ES FR GB IT LI**

(30) Priorität: **20.10.1992 DE 4235295**

(43) Veröffentlichungstag der Anmeldung:
**09.08.1995 Patentblatt 1995/32**

(73) Patentinhaber: **Degussa Aktiengesellschaft
60311 Frankturt (DE)**

(72) Erfinder:
- **HASSEBERG, Hans-Albrecht
D-63517 Rodenbach (DE)**
- **HUTHMACHER, Klaus
D-63571 Gelnhausen (DE)**
- **RAUTENBERG, Stephan
D-63457 Hanau (DE)**
- **PETSCH, Heinrich
D-63456 Hanau (DE)**
- **WEIGEL, Horst
D-63517 Rodenbach (DE)**

(56) Entgegenhaltungen:
**EP-A- 0 084 470** **EP-A- 0 228 938**
**FR-A- 2 405 924**

EP 0 665 832 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung umfaßt ein vorzugsweise kontinuierlich durchführbares Verfahren, das zur großtechnischen Herstellung von wäßrigen Alkali- oder Erdalkalimethioninatlösungen geeignet ist, die direkt z. B. als Futtermittelzusatz verwendet oder auch zur Isolierung von als Futtermittelzusatz verwendbaren Aminosäuren oder deren Salzen eingesetzt werden können. Das Verfahren ist insbesondere zur Herstellung von Methioninatlösung geeignet. Methionin sowie wäßrige Lösungen von Methioninsalzen, insbesondere von Natriummethioninat (DE 31 05 009 C), aber auch Ersatzstoffe wie das Methionin-Hydroxyanaloge (MHA) werden weltweit als Futtermittelzusatz für die Aufzucht von Geflügel, Schweinen und anderen Nutztieren verwendet und kommen hauptsächlich der Produktion von tierischem Eiweiß zugute. Gerade im Hinblick auf die steigende Weltbevölkerung und die zunehmenden Ernährungsprobleme kommt dem Methionin als einer der erstlimitierenden Aminosäuren im tierischen Wachstumsprozeß und seinen verschiedenen Formen und damit auch deren kostengünstiger Herstellung eine besondere Bedeutung zu. Je nach Erfordernissen werden Feststoff oder Flüssigformen bevorzugt eingesetzt.

Die im Handel befindliche Natriummethioninat-Lösung hat eine Konzentration von 40 Gew.-% Methionin und entspricht, im Gegensatz zum Ersatzstoff HHA, hinsichtlich ihrer biologischen Wertigkeit dem festen Methionin, verglichen auf äquimolarer Basis. Für die Herstellung solcher Natriummethioninat-Lösungen kommen hauptsächlich 3 Methoden in Betracht:

1. Einfaches Auflösen von isoliertem Methionin.

Diese Methode liefert zwar die reinste Produktform, ist aber durch einen zusätzlichen Arbeitsschritt im Vergleich zur Feststoffproduktion aufwendiger und damit weniger wirtschaftlich als die Herstellung von Methionin selbst.

2. Die alkalische Hydrolyse von

5-(β-Methylmercaptoethyl)-hydantoin mit NaOH oder NaOH/$Ca(OH)_2$-Gemischen, bei der, um unerwünschte Nebenproduktbildung zu vermeiden, ca. 2 - 3 Hydroxidäquivalente eingesetzt werden müssen.

Bei Verwendung von $Ca(OH)_2$ kann der Überschuß an Verseifungsagens zwar (DE 31 05 006 C) in Form von Calciumcarbonat abgetrennt werden, muß jedoch als kaum verwertbares Abfallsalz entsorgt oder durch aufwendige Zusatzschritte wie Brennen und Löschen in die wiedereinsetzbare Hydroxidform zurückgeführt werden. Bei alleiniger Verwendung von Natronlauge hingegen muß der Überschuß entweder (DE 31 04 997 A) in Form des gebildeten Natriumcarbonats oder (EP 253 740) nach Neutralisation des Natriumcarbonats mit Schwefelsäure in Form des dann gebildeten Natriumsulfats entfernt werden. Der Salzanfall, sowie die aufwendige Salzabtrennung, aber auch die Gefahr zurückbleibender unerwünschter Salzreste in der Produktlösung bilden die Nachteile dieser Verfahrensweisen.

3. Die alkalische Hydrolyse von Methioninamid.

Diese kann, wie z. B. aus EP 0 228 938 bekannt, mit in etwa stöchiometrischen Mengen Hydroxid durchgeführt werden, ohne daß es zu größerer Nebenproduktbildung kommt. Hierin liegt ein wesentlicher Vorteil gegenüber der Verfahrensweise gemäß 2.

Die Herstellung von Methioninamid kann in bekannter Weise durch Hydrolyse von Methioninnitril geschehen, welches wiederum durch Direktsynthese aus den üblichen Ausgangsstoffen Methylmercaptopropionaldehyd (MMP), Blausäure oder Ammoniumcyanid und Ammoniak gewonnen werden kann.

Die sauer katalysierte Nitrilhydrolyse scheidet wegen des hier zwangsläufig auftretenden Neutralsalzanfalls aus, während die alkalische Nitrilhydrolyse vorzugsweise unter Zusatz katalytisch wirksamer Carbonylverbindungen, insbesondere von Ketonen durchgeführt wird (Houben-Weyl: Methoden der Organischen Chemie, Erweiterungs- und Folgebände zur

4. Aufl. 1985, 8d. E5, S. 535ff. und die dort zitierte Literatur).

Da es relativ aufwendig und verlustreich ist,

Methioninamid sauber zu isolieren um es anschließend, wie beispielsweise im Patent EP 228 938 beschrieben, mit NaOH zum NaMet zu hydrolysieren, ist ein solches Verfahren unwirtschaftlich.

Im anderen angegebenen Beispiel des gleichen Patents wird in einem 500 ml-Druckbehälter aus MMP und HCN zunächst in üblicher Weise MMP-Cyanhydrin gebildet und dieses durch anschließendes Einbringen eines Überschusses an kondensiertem $NH_3$ zum Methioninnitril umgesetzt. Nach Entspannen und Abkühlung von 60°C auf 10°C wird dieses (vgl. Houben-Weyl) mit einer wäßrigen Lösung von Aceton und NaOH umgesetzt und anschließend durch Anlegen von Vakuum restlicher Ammoniak und Aceton entfernt, bevor das gebildete Methioninamid nach Zugabe von

Natronlauge bei Temperaturen von 180°C zum Natriummethioninat hydrolysiert wird.

Anschließendes Entspannen und Entfernen von Restammoniak im Vakuum führt zu einer Natriummethioninatlösung, die neben Methionin noch mind. 10 weitere Nebenprodukte in unterschiedlicher, aber nicht vernachlässigbarer Konzentration enthält. Diese labortechnische Ausführungsform ist für den Einsatz im Bereich üblicher großtechnischer Produktionen von mehreren 10 000 jato Methionin pro Anlage ungeeignet. Ferner wird über den Verbleib des Acetons bzw. seine Recyclierbarkeit, ebenso über die Recyclierbarkeit des eingesetzten großen $NH_3$-Überschusses keine Aussage gemacht.

Eine möglichst vollständige Rückgewinnung des im Überschuß eingesetzten Ammoniaks sowie des benötigten Keton-Katalysators in recyclierbarer Form und anschließende Wiederverwendung von beiden sind jedoch, neben der optimalen Gestaltung der hier gewählten Syntheseroute hinsichtlich Ausbeute und Selektivität, Voraussetzung für ein ökonomisches Verfahren, welches zudem in der angestrebten Größenordnung kontinuierlich ausführbar sein sollte.

Bisher ist noch nie ein kontinuierlicher Prozeß zur homogen Keton-katalysierten alkalischen Aminonitrilhydrolyse beschrieben worden. Der Grund dafür dürfte darin bestehen, daß bisher keine wirksame und kontinuierlich durchführbare technische Reinigungsmethode für das anfallende Gemisch aus Keton, Ammoniak, Wasser sowie ggf. aus Nebenprodukten zur Verfügung stand.

Die Bedeutung dieses Problems läßt sich daran ablesen, daß man als Ketonersatz katalytisch aktive carbonylgruppentragende Polymerharze entwickelt hat (EP 84 470, DD 208 349), die aus dem Reaktionsmedium leicht durch Filtration abgetrennt oder in Form eines Festbetts von der Substratlösung kontinuierlich durchströmt werden können.

Dieses auf den ersten Blick sehr attraktive Konzept hat jedoch den Nachteil, daß solche Katalysatoren bisher nicht käuflich sind und bei einer etwaigen Herstellung in den für die Großproduktion dann notwendigen Mengen sehr teuer sein dürften, im Gegensatz zu den käuflichen preiswerten Ketonen. Zudem werden die beschriebenen Polymerharze schnell durch Nebenreaktionen an den Carbonylgruppen vergiftet und müssen aufwendig regeneriert werden.

Bei einer Variante dieses Verfahrens (EP 168 282, US 4,677,224) wird eine Verlängerung der Katalysator-Standzeit mit einer starken Rückverdünnung der zur Hydrolyse eingesetzten Aminonitrillösung von ca. 1 M auf 0,1 M durch die in der nachfolgenden Verseifungsstufe hergestellte Produktlösung erkauft.

Dies ist für eine Großproduktion jedoch ebenfalls unvorteilhaft. Zum einen werden entsprechend größere mit Polymerkatalysator gefüllte Säulen gebraucht, die mindestens 2mal vorhanden sein müssen. wobei die Katalysatorregenerierung trotzdem nicht ganz entfällt. Zum anderen müssen permanent ca. 90 % der ca. 80°C heißen Produktlösung wieder auf die in der Vorstufe nötige Temperatur von 30°C heruntergekühlt werden, um sie nach Durchlaufen der Stufe wieder auf die ursprüngliche Verseifungstemperatur aufzuheizen.

Eine Vermeidung bzw. Minimierung von Abfallströmen sowie eine optimale Prozeßführung sind sowohl unter ökonomischen, als auch unter modernen ökologischen Gesichtspunkten in einem solchen Prozeß ebenfalls unerläßlich.

Aufgabe ist daher ein Verfahren zur Herstellung von Methionin oder dessen Salz oder Vorstufen wie Methioninnitril oder -amid, wobei das Verfahren direkt von den einzusetzenden bereits genannten Grundchemikalien ausgeht und auch ohne Isolierung der Zwischenstufen großtechnisch durchführbar sein soll. Das Verfahren soll wirtschaftlich und insbesondere kontinuierlich durchführbar sein, wobei der überschüssige Ammoniak, sowie das eingesetzte Keton möglichst weitgehend verlustfrei zurückgewonnen und wiedereingesetzt werden sollen.

Diese Aufgabe wird durch das vorliegende Verfahren gemäß der Ansprüche gelöst, wobei insbesondere die Kombination der Ansprüche unter Erhalt der jeweiligen Vorteile möglich ist.

Das vorzugsweise kontinuierlich, oder teilweise kontinuierlich aber auch diskontinuierlich durchführbare Verfahren umfaßt insgesamt 5 oder 6 Hauptverfahrensschritte:

1.) Bildung von Roh-MMP-Cyanhydrin (CH) aus MMP und HCN (Vorstufe)

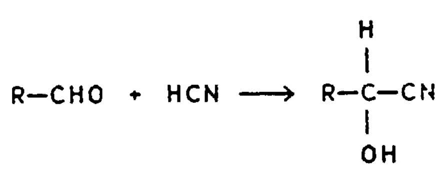

2.) Bildung von Roh-MMP-Aminonitril aus Roh-MMP-CH und $NH_3$ oder $NH_3$/Wasser-Gemischen oder in einem Schritt aus MMP, HCN und $NH_3$ bzw. $NH_3$/Wasser-Gemischen (Vorstufe)

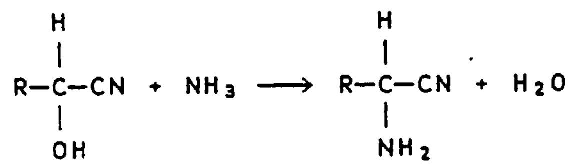

oder

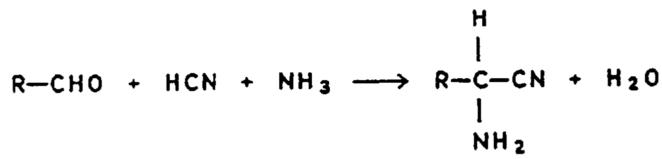

3.) Alkalische Keton-katalysierte Hydrolyse von Roh-MMP-Aminonitril (AN) zum Roh-MMP-Amid

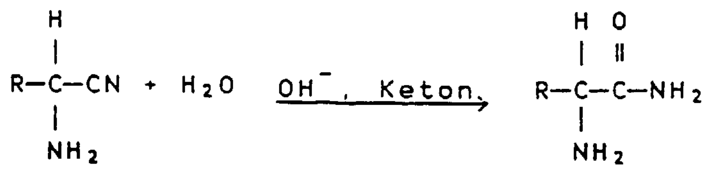


4.) Alkalische Verseifung des Methioninamid (Met-AM) enthaltenden Rohproduktgemisches aus 3.) ggf. unter gleichzeitiger teilweiser oder vollständiger Abtrennung von Keton, $NH_3$ und ggf. weiterer flüchtiger Komponenten

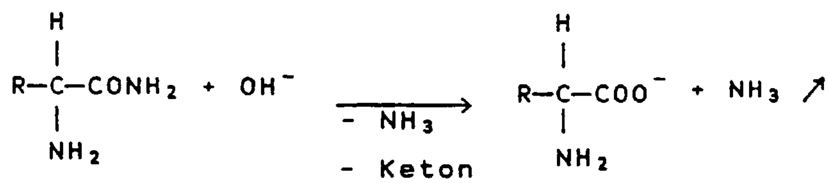


5.) Ggf. Nachbehandlung der aus 4.) erhaltenen Roh-Methioninat-Lösung, wie z. B. Nachreaktion, Reinigungsschritt bzw. Eindampfung bis zur gewünschten Endkonzentration.

6.) Ammoniak/Keton-Reinigung und Recyclierung

$$R = (CH_2)_2SCH_3$$

Zu 1.) und 2.):

Die Bildung von Aminonitrilen als α-Aminosäurevorstufen aus Aldehyden mit Blausäure und Ammoniak (Streckor-Synthese) oder aus primär gebildeten Cyanhydrinen und Ammoniak (Tiemann-Variante) ist allgemein bekannt und an zahllosen Beispielen untersucht worden (Houben-Weyl: Methoden der Organischen Chemie, 4. Aufl., 1952, Bd. 8, S. 274ff und S. 279ff bzw. Erweiterungs- und Folgeband zur 4. Aufl. E5 S. 1425ff). Beide Reaktionen laufen bei geeigneter Wahl der Reaktionsbedingungen mit > 90 % Ausbeute ab.

Die Tiemann-Variante hat im vorliegenden Fall den Vorteil, daß die relativ große Reaktionswärme der Cyanhydrin-Bildung zunächst abgefangen werden kann. Das ermöglicht insbesondere bei kontinuierlicher Fahrweise eine exaktere Einstellung der Reaktionstemperatur der nachfolgenden ebenfalls exothermen Aminonitril-Bildung.

**MMP-Cyanhydrin-Bildung:**

Die Bildung von MMP-Cyanhydrin erfolgt spontan aus MMP und flüssiger oder gasförmiger Blausäure bei pH 5 - 9 und Temperaturen bis 50°C, wird jedoch vorzugsweise bei pH 5,5 - 7,5 und Temperaturen zwischen 20 und 30°C durchgeführt.

Der pH-Wert wird ggf. durch Zusatz geringer Mengen an Base im Sollbereich gehalten. Geeignet sind sowohl organische Basen, wie Aminverbindungen, beispielsweise Triethylamin oder Pyridin, aber auch anorganische Basen wie Alkali- oder Erdalkalihydroxide bzw. -cyanide wie beispielsweise NaOH oder NaCN, insbesondere in Form ihrer wäßrigen Lösungen.

Die Reaktionspartner sollten in etwa equimolar eingesetzt werden. Ein Aldehydüberschuß sollte möglichst vermieden werden, da er zu Neben- und Abbauprodukten in den folgenden Reaktionsstufen bzw. im Endprodukt führt.

Ein großer HCN-Überschuß sollte ebenfalls vermieden werden, da die Blausäure entweder an geeigneter Stelle wieder ausgetrieben oder zerstört werden muß, wodurch ein zusätzlicher Energie- bzw. verfahrenstechnischer Aufwand entstehen würde. Ein geringfügiger HCN-Überschuß von bis zu 5 Mol % insbesondere, hat sich hier jedoch als vorteilhaft erwiesen, da dadurch ein quantitativer MMP-Umsatz mit praktisch $\geq$ 99,9 %-iger Selektivität erreicht werden kann.

Die MMP-CH-Herstellung kann aus MMP und gasförmiger oder flüssiger Blausäure sowohl im batch, beispielsweise in einem Rührkessel, als auch besonders vorteilhaft kontinuierlich in einem Rohr- oder Schlaufenreaktor oder in einer Kombination aus beiden in der oben erläuterten Weise durchgeführt werden. Die gute Durchmischung der Reaktanden bei gleichzeitiger pH- und Temperaturkontrolle sind Voraussetzung für eine optimale Durchführung dieses Verfahrensschrittes. Die mit üblichen Analysenmethoden, wie HPLC oder DC nachgewiesenen Ausbeuten lagen bei 99,9 %.

**Aminonitril-Bildung:**

Das erhaltene Roh-MMP-Cyanhydrin kann ebenso wie in etwa equimolare Mischungen aus MMP und HCN oder ein beliebiges Gemisch aus Roh-MMP-Cyanhydrin und MMP/HCN, wie es beispielsweise bei nicht vollständig gebildetem MMP-CH in der Cyanhydrin-Stufe entsteht, mit Ammoniak oder Ammoniak/Wasser-Mischungen zum Roh-MMP-Aminonitril umgesetzt werden.

Während in älteren Patentveröffentlichungen vor allem Umsetzungen mit flüssigem $NH_3$ und MMP/HCN bzw. MMP-CH beschrieben sind, wurde in jüngerer Zeit die vorteilhafte Verwendung von wäßrigem Ammoniak auf die Aminonitril-Bildung hervorgehoben.

In DE-OS 16 43 535 wird die Verwendung von $NH_3$-Überschüssen von 4,6 - 55 eq bez. auf MMP und $NH_3$-Konzentrationen von > 50 Gew.% unter Überdruck bei Temperaturen bis max. 60°C und vorzugsweise 15 - 40°C beschrieben. Die Verweilzeiten sind jedoch mit 4,5 bis 5,5 Std. insbesondere für ein kontinuierliches Verfahren zu lang. Außerdem entstehen bei der beschriebenen Verfahrensweise zuviel Nebenprodukte (DN und DNMA), die für eine kontinuierliche Großproduktion ebenfalls nicht akzeptabel sind. Die Methioninausbeuten nach anschließender saurer Hydrolyse des Aminonitrils werden zwischen 97 und 99 % bez. auf MMP angegeben. DE-OS 20 06 979 lehrt ein in den wesentlichen Bedingungen gleich verlaufendes Verfahren, das jedoch in kontinuierlich betriebenen Rührkesseln durchgeführt wird, wobei der Schwerpunkt dieser Erfindung auf der teilweisen Rückführung einer nach Phasentrennung am Rohaminonitril erhaltenen NH3-reichen und noch AN-haltigen Wasserphase in den Reaktionskessel liegt. Dadurch soll der Salzanfall bei der anschließenden sauren Verseifung verringert werden. Die teilweise Rückführung bereits gebildeten Produktes schränkt den wirtschaftlichen Wert dieser Verfahrensweise jedoch stark ein, insbesondere auf die dort angestrebte saure Verseifung.

In DE 26 45 544 wiederum wird die Verwendung von wäßrigem Ammoniak mit $NH_3$-Konzentrationen von 24 - 48,6 Gew.%, insbesondere von 32 bis 42 Gew.% und Überschüssen von 4 - 7 eq $NH_3$ bez. auf das dort eingesetzte vorgebildete Cyanhydrin bei Temperaturen zwischen 50 und 100°C und Drücken von 1 - 10 bar als vorteilhaft beschrieben. Die Aminonitrilbildung kann zwar kontinuierlich und in einem röhrenförmigen Reaktor betrieben werden und die Reaktionszeiten liegen unter 30 min, die Aminonitril-Ausbeute erreicht jedoch im besten Fall nur 96 % bez. auf eingesetztes MMP-Cyanhydrin. Eine Zwischenausbeute von deutlich unter 100 % ist jedoch für ein mehrstufiges Verfahren, sowohl unter ökonomischen wie unter ökologischen Gesichtspunkten nicht ausreichend, da davon auszugehen ist, daß unumgesetztes Edukt oder daraus entstandene Nebenprodukte abgetrennt und aufwendig entsorgt werden müssen. Dies ist insbesondere bei Größenordnungen, wie sie für die Methioninproduktion üblich sind, untragbar.

In allen zitierten Patentveröffentlichungen werden zwar Ausbeuten über 90 % angegeben, über die in der Bilanz auftretende Lücke wird aber keine Aussage gemacht.

Erfindungsgemäß wurde nun gefunden, daß die organischen Hauptnebenkomponenten im Rohaminonitril aus dem entsprechend substituierten Iminodinitril (DN) (DN = 2,2'-Bis-(2-methylmercaptoethyl)-iminodiacetonitril), dem davon abgeleiteten Dinitrilmonoamid (DNMA) (DNMA 2,2'-Bis-(2-methylmercaptoethyl)-iminodiacetonitrilmonoamid), sowie bereits an dieser Stelle gebildetem Methioninamid (AM) bestehen.

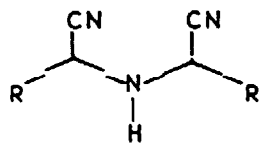

DN

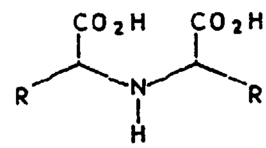

DS 1

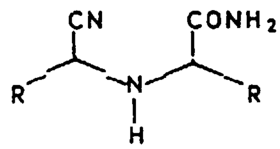

DNMA

R mit obiger Bedeutung.

Die bisher noch nicht in der Literatur beschriebenen Iminodinitrilverbindungen DN und DNMA hydrolysieren dann in den Folgestufen ganz oder teilweise zur entsprechenden Iminodisäure (DS1 = 2,2'-Bis(2-methylmercaptoethyl)-iminodiessigsäure), deren Gehalt als Fremdkomponente im Produkt möglichst niedrig liegen sollte. Folglich kommt es im Interesse einer optimalen Ausführungsform der Aminonitrilstufe in erster Linie darauf an, bei praktisch quantitativer Aminonitril-Ausbeute die Nebenproduktbildung so gering wie möglich zu halten, insbesondere unter Berücksichtigung der für ein wirtschaftliches Verfahren wichtigen Aspekte.

Es wurde nun gefunden, daß der DN+DNMA-Anteil einen vertretbar kleinen Wert von ≤ 2 Mol % des eingesetzten Cyanhydrins annimmt bei AN-Ausbeuten von ≥ 98 Mol %, jeweils bezüglich des umgesetzten Aldehyds, wenn man mit einem molaren Verhältnis von $NH_3$/CH bzw. $NH_3$/Aldehyd von mindestens 4 : 1, insbesondere 5 : 1 bis 15 : 1 und eingesetzten $NH_3$-Konzentrationen von > 50 Gew.-%, vorteilhaft 55 - 85 Gew.-% und insbesondere über einem Molverhältnis $NH_3/H_2O$ von 1,5, bei Temperaturen über 40°C jedoch nicht über 80°C und bei Reaktionszeiten von mindestens 5 jedoch nicht über 60 min arbeitet. Insbesondere hohe Temperaturen oder sinkende $NH_3$-Konzentrationen bzw. -überschüsse, aber auch lange Verweilzeiten lassen den Dinitrilanteil ansteigen, während niedriger Wassergehalt die nötige Reaktionszeit verlängert. Vorzugsweise wird die Reaktion spätestens dann beendet, wenn 2 % d. Th. (mol-% bez. MMP+CH) an DN+DNMA entstanden sind, da sonst der DS-Anteil im Endprodukt zu hoch wird. Im allgemeinen wird die Reaktion innerhalb der Grenzen so geführt, daß der DN+DNMA-Anteil möglichst gering ist. Eine bevorzugte Ausführungsform des vorliegenden Verfahrensschrittes ist die Verwendung von 5 - 8 eq $NH_3$ mit einer Konzentration von 55 - 85 Gew.-%, insbesondere 60 - 80 Gew.-% im Temperaturbereich zwischen 50 und 70°C bei Reaktionszeiten von 10 bis 50 min und dem sich dabei einstellenden Systemdruck. Innerhalb dieses Bereichs läßt sich der DN+DNMA-Anteil sogar auf < 1 Mol % absenken.

Die hier gewählten Bedingungen ermöglichen erstmals eine kontinuierliche Fahrweise mit Ausbeuten über 96 %, die für eine Großproduktion unabdingbar ist. Für das Verfahren kann ein reiner Rohrreaktor oder eine Kombination von Schlaufen- und Rohrreaktor eingesetzt werden, wobei der Rohrreaktor ein Mehrfaches der Verweilzeit des Schlaufenreaktors aufweisen sollte, da dadurch die Dinitrilbildung kleiner gehalten werden kann. Das hier beschriebene Verfahren zeichnet sich zudem dadurch aus, daß anstelle von frischem Ammoniak/Wasser der aus den späteren Recyclierungsstufen zurückgewonnene Ammoniak bzw. entsprechende Ammoniak/Wasser-Gemische verwendet werden können.

Die auf diese Weise gewonnen Rohaminonitril/Wasser/Ammoniak-Gemische können entweder direkt für die nachfolgende Hydrolyse oder nach Entspannen auf Normaldruck oder einem Druck, der zwischen dem in der Aminonitrilstufe herrschenden Systemdruck und Normaldruck liegt, eingesetzt werden. Bei Entspannung kann bereits an dieser Stelle ein Teil des überschüssigen $NH_3$ aus der Aminonitrilstufe zurückgewonnen werden.

**Aminonitril-Hydrolyse = Methioninamid-Bildung:**

Die in den Patenten DE 26 37 204 und DE 27 53 828 zur Herstellung von Aminosäureamiden durch Keton-katalysierte Hydrolyse eines entsprechenden Aminonitrils vorgeschlagenen aliphatischen und cycloaliphatischen Ketone zeigen deutliche Unterschiede in ihrer Eignung für die Hydrolyse des Aminonitrils.

Die größte Reaktionsgeschwindigkeit erreicht man mit Aceton dem preiswertesten, am wenigsten gesundheitsgefährdensten und damit für industrielle Zwecke vorzugsweise einzusetzenden Keton.

Die Hydrolyse von wie beschrieben gebildetem Aminonitril mit verdünnter Natronlauge in Gegenwart von 0,2 bis 2,0 eq Aceton bei Temperaturen zwischen 10 und 30°C führt zu einem Produktgemisch, das in der Hauptsache aus Methioninamid, Natriummethioninat, den Imidazolidinonverbindungen IM1 und IM2, sowie MMP und weiteren in kleineren Konzentrationen auftretenden Komponenten besteht.

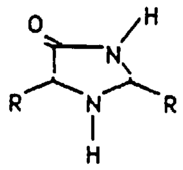

IM1

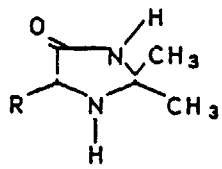

IM2

R mit obiger Bedeutung.

Die Imidazolidinone werden zwar in der nachfolgenden Verseifung bei höherer Temperatur auch in Methionin umgewandelt, insbesondere der IM1- und MMP-Anteil führen jedoch zu Verlusten in der Endausbeute und ggf. zu Reinheitsproblemen. Erfindungsgemäß kann die Aldehyd-Rückbildung und die IM1-Bildung durch Niedrighalten der Temperatur und des Alkaligehalts deutlich reduziert und dadurch die Selektivität der Gesamtreaktionsfolge erhöht werden. Ein Verfahren, wie es im Patent DE 27 53 829 zur Herstellung von α-Aminosäuren beschrieben wird, bei dem die gesamte zur Aminonitrilverseifung nötige Alkalimenge, also im wesentlichen 1 eq Hydroxid, bereits dem Rohaminonitrilgemisch zugesetzt und die Verseifung in einem Schritt durchgeführt wird, führt im vorliegenden Fall zu eher schlechteren Ergebnissen. Ebenso kann bei einer Temperaturerhöhung auf über 40°C ein Anstieg des Aldehyd-Anteils auf bis zu 7 Mol % beobachtet werden. Der Aldehyd-Anteil kann jedoch bei Temperaturen unter 30°C auf unter 2 Mol % abgesenkt werden. Da die Reaktionsgeschwindigkeit der Aminonitrilhydrolyse bekanntermaßen stark mit der Ketonkonzentration steigt (Commeyras et al., Tetrahedron 1978, 34, 2275ff am Beispiel von Aceton und α-Alaninnitril), kann auch diese Eigenschaft zur gezielten Reaktionssteuerung ausgenutzt werden. Ein zu großer, wie beispielsweise mehrfach molarer, Keton-Überschuß ist jedoch aus Gründen der Wirtschaftlichkeit und der Produktreinheit nicht von Interesse. Es wurde nun gefunden, daß innerhalb eines $H_2O$-Überschußbereichs bei moderatem Einsatz von Aceton und vorzugsweise niedrigen Mengen an Hydroxid hohe Reaktionsgeschwindigkeiten und damit einhergehend sehr gute Selektivitäten in der AN-Hydrolysestufe erreicht werden können. Zusammenfassend läßt sich der im vorliegenden Falle optimale Bereich folgendermaßen eingrenzen (bezogen auf das Nitril):

15 - 50 eq, vorzugsweise 20 - 30 eq $H_2O$; 0,2 bis 2,0 eq, vorzugsweise 0,3 - 1,0 eq Keton; 0,1 - 1,1 eq, vorzugsweise 0,15 - 0,4 eq und insbesondere 0,15 - 0,3 eq OH- und 1 - 7 eq, vorzugsweise 2 - 5 eq $NH_3$ bei Temperaturen zwischen 10 und 30°C, vorzugsweise von 15 - 28°C und Reaktionszeiten zwischen 10 und 90 min, vorzugsweise von 20 - 60 min. Das hier beschriebene Verfahren zeichnet sich insbesondere dadurch aus, daß anstelle von Keton und Wasser eine in einer später folgenden Recyclierungsstufe zurückgewonnene wäßrige Ketonlösung, insbesondere Acetonlösung inklusive darin enthaltener Nebenkomponenten verwendet werden kann.

Ebenso wie bei den vorhergehenden Verfahrensschritten läßt sich die Hydrolyse von Rohaminonitril sowohl diskontinuierlich als auch vorzugsweise kontinuierlich realisieren, im ersten Falle z. B. in einem gerührten Reaktionsgefäß. Im zweiten Falle kann ein einfaches Reaktionsrohr, besser aber ein Schlaufenreaktor, insbesondere eine Kombination von Schlaufenreaktor und Reaktionsrohr zum Einsatz gebracht werden. Der Druck in dieser Stufe ist unkritisch und kann je nach Verfahrensweise frei gewählt werden. Vorzugsweise wird jedoch der sich bei gegebener Verfahrensweise einstellende Systemdruck angewendet.

**Aminosäureamid-Hydrolyse = Hethioninat-Bildung:**

Die so erhaltene Lösung kann direkt für die nachfolgende Verseifung mit Alkali- bzw. Erdalkalihydroxid eingesetzt werden. Dazu wird der bereits in der vorhergehenden Hydrolysestufe eingestellte Hydroxid-Gehalt durch Zugabe weiterer Hydroxidäquivalente erhöht, so daß der resultierende Hydroxid-Gehalt einem Verhältnis von 0,95 bis 1,1 eq, vorzugsweise 1,0 bis 1,05 eq bezogen auf das eingesetzte MMP entspricht. Die Verseifungstemperatur sollte mindestens 85°C, vorzugsweise mindestens 90°C, in der Regel jedoch über 100°C, insbesondere 110 - 140°C betragen. In der Regel werden 200°C nicht überschritten. Die Amidkonzentration beträgt vorteilhaft 10 bis 35 Gew.-%, vorzugsweise ≤ 30 Gew.-% und insbesondere ≤ 25 Cew.-%, wobei bei einer Verseifungstemperatur ≥ 160°C eine Konzentration ≤ 25 Gew.-% bevorzugt ist. Als Druck wird der zur gewählten Temperatur korrespondierende Systemdruck von 1 bis mehreren bar eingestellt, ggf. auch mit gleichzeitiger Eindampfung der Reaktionslösung verbunden. Die Reaktionsdauer beträgt mindestens 10, aber maximal 90 min, vorzugsweise 20 - 40 min im Falle von Alkalimetallhydroxid bzw. bis zu 180 min im Fall von Erdalkalimetallhydroxid.

Das erfindungsgemäße Verfahren zeichnet sich dadurch aus, daß während und/oder nach der Verseifung des

Amids Ammoniak, Keton und Wasser gemeinsam bei einer Temperatur ≥ 85°C und/oder unter Anlegen eines Vakuums abgezogen werden. Der Ammoniak wird vorteilhaft vom Keton im wesentlichen befreit und der Nitrilsynthese (s. o.) zugeführt. Das erfindungsgemäße Verfahren kann bei allen Methioninsynthesen eingesetzt werden, bei denen Methioninnitril in Gegenwart eines Ketons zuerst zum Amid, insbesondere bei einer Temperatur unter 60°C, verseift wird.

Erfindungsgemäß wurde festgestellt, daß es keineswegs notwendig ist das im Rohmethioninamid-Gemisch enthaltene Keton vorher abzutrennen, wie es z. B. im EP-B 228 938 durch Anlegen von Vakuum an die hergestellte Methioninamidlösung erreicht und in der DE 26 37 204 C2 (wegen des niedrigen pH) vorgeschlagen wird. Da ein Teil des Ketons (2 - 10 % der eingesetzten Menge) - wie erfindungsgemäß festgestellt wurde - ohnehin als Imidazolidinon (IM2 im Falle von Aceton) in chemisch gebundener Form vorliegt und erst in der Verseifungsstufe freigesetzt wird, ist aus ökonomischen wie ökologischen Gründen hier eine Rückführung notwendig und die gemeinsame Rückführung von freiem und freigesetztem Keton in einem während bzw. nach der Amid-Verseifung die Methode der Wahl. Vorzugsweise wird vor dem Amid-Verseifungsschritt kein Keton abgezogen, ein zweiter Teilstrom mit Keton-haltigen Brüden und der damit einhergehende apparative Mehraufwand wird auf diese Weise vermieden. Die Verseifungsstufe kann sowohl diskontinuierlich in einem gerührten Druckreaktor, besser jedoch kontinuierlich z. B. in einer Verseifungskolonne durchgeführt werden. Bei der kontinuierlichen Fahrweise ist es besonders vorteilhaft gleichzeitig das restliche und das bei der Reaktion gebildete $NH_3$ zusammen mit dem noch vorhandenen Keton und dem bei der Verseifungstemperatur in situ erzeugten Wasserdampf oder dem wahlweise zusätzlich eingeblasenen Heizdampf zu entfernen. Dieses über Kopf entweichende Gemisch wird aufgefangen und zur Ammoniak- und Ketonrecyclierung eingesetzt. Der Ablauf der Kolonne enthält dann eine ammoniak- und ketonfreie, je nach Eindampfungsgrad unterschiedlich konzentrierte hellgelbe Aminosäuresalzlösung mit einem Aminosäuregehalt von 10 bis 45 Gew. %.

Da in den Rohaminosäuresalzlösungen noch Cyanidreste in Konzentrationen von mehreren hundert ppm auftreten können, ist es notwendig diese ggf. zu zerstören. Erfindungsgemäß erfolgt dies durch thermische Behandlung vorzugsweise der unmittelbar erhaltenen Aminosäuresalzlösungen bei über 150°C bis vorzugsweise 200°C und Verweilzeiten bis 40 min, insbesondere von mind. 10 min. Im allgemeinen gilt, daß bei ≥ 160°C mind. 15 min, bei ≥ 180°C mind. 7 min und bei ≥ 190°C mind. 4 min behandelt wird.

Aus der DE-OS 33 34 328 ist es zwar bekannt, daß restliche Cyanidkonzentrationen durch Temperaturerhöhung in alkalischer Lösung zerstörbar sind, die DE-OS 33 34 328 betrifft allerdings die Vernichtung von gebundener Blausäure und Acrylnitril in rohem Acetonitril, wobei deutliche andere Temperatur und pH-Werte, als bei der Umsetzung von Aminosäuren zu berücksichtigen sind.

Die so erhaltenen Lösungen weisen nur noch einen Cyanidgehalt von < 10 ppm, bezogen auf 40 Gew.% Aminosäuregehalt auf. Dies bedeutet eine deutliche Unterschreitung des zulässigen Grenzwertes. Die thermische Nachbehandlung kann bei jeder der erhaltenen Aminosäurekonzentrationen von 10 - 45 Gew.%, vorzugsweise jedoch bei 15 - 30 Gew.%, erfolgreich durchgeführt werden. Sie kann sowohl in einem nachgeschalteten Schritt, beispielsweise einem zusätzlichen Reaktionsrohr, aber auch während der eigentlichen Verseifung, also z. B. durch entsprechende Temperaturerhöhung im unteren Teil der Verseifungskolonne durchgeführt werden. Die Cyanidverseifung führt zu einem geringfügigen Anteil an Alkali- ggf. Erdalkaliformiat in der Produktlösung, welches jedoch keineswegs stört, da Formiat, insbesondere Calciumformiat selbst als Futtermitteladditiv verwendet wird.

Vor allem bei Ablaufkonzentrationen von < 40 Gew.% Aminosäure ist eine zusätzliche Eindampfung erforderlich. Diese wird vorteilhaft bei Normaldruck oder reduziertem Druck und ggf. Siedetemperatur unter möglichst schonenden Bedingungen durchgeführt, beispielsweise unter Einsatz eines Fallfilmverdampfers. Die hier erhaltenen Brüden können an anderer Stelle wieder in den Prozeß zurückgeführt werden.

Es hat sich auch als günstig herausgestellt, die Produktlösung zu filtrieren, vorzugsweise über Aktivkohle. Dies kann insbesondere bei kontinuierlicher Fahrweise vorteilhaft in einer Kolonne durchgeführt werden. Neben der Entfernung von zwar nur geringfügigen Trübungen, sowie kleinen Nebenproduktanteilen tritt hier eine weitere Farbaufhellung ein. Dieser Schritt kann sowohl vor als auch nach einer ggf. durchzuführenden Eindampfung stattfinden. Die Gesamtausbeute an Methionin liegt bei der hier gezeigten Verfahrensweise bei mindestens 95 % bezogen auf eingesetzten Aldehyd. Der Gehalt der in der Lösung nachweisbaren untoxischen Iminodiessigsäurekomponenten DS1 und DS2 (N-(1-Carboxy-1-methylethyl)-methionin) liegt bei

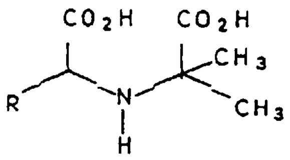

DS2

R mit obiger Bedeutung

kontinuierlicher Verseifung in Summe unter 1 Gew. % bezogen auf Methionin. Die Qualität der Produktlösungen entspricht der im Futtermittelbereich eingesetzten Qualität.

**Ammoniak/Keton-Reinigung und Recyclierung:**

Für die Wirtschaftlichkeit dieses Verfahrens ist es von ausschlaggebender Bedeutung, den überschüssigen Ammoniak wiederzuverwerten, insbesondere zurückzuführen in den Aminonitril-Bildungsteil. Da im Laufe der Aminonitrilverseifung wieder ein $NH_3$-Äquivalent entsteht, ist es obendrein bei erfolgreicher Recyclierung sogar möglich, den gesamten Prozeß ohne $NH_3$-Verbrauch durchzuführen. Der im Aminosäuremolekül enthaltene Stickstoff stammt somit letztlich aus der Blausäure.

Ebenfalls aus Gründen der Wirtschaftlichkeit ist eine möglichst vollständige Recyclierung des als Katalysator eingesetzten Ketons notwendig.

Erfindungsgemäß hat sich gezeigt, daß der zur Aminonitrilbildung eingesetzte Ammoniak im wesentlichen ketonfrei sein muß, da bereits kleine Anteile zu einer verringerten Aminonitrilausbeute und einem deutlich meßbaren Aldehyd-Anteil im Rohaminonitril und damit schließlich zu Ausbeuteverlusten und Qualitätseinbußen im Endprodukt führen. Der recyclierte Ammoniak soll vorzugsweise weniger als 25 meq und insbesondere weniger als 10 meq Keton bezüglich des Cyanhydrins und/oder des Aldehyds enthalten. Das bedeutet folglich, daß die abgestrippte $NH_3$/Keton/Wasser-Mischung nicht direkt recycliert werden kann, wobei hier unerheblich ist, ob vor, während oder nach der Amid-Verseifung abgestrippt wird - die beiden letzteren Alternativen sind, wie ausgeführt, jedoch bevorzugt. Vielmehr muß zunächst durch eine geeignete Trennoperation in möglichst quantitativer Ausbeute im wesentlichen ketonfreier NH3 gewonnen werden, während das zu recyclierende Keton sowohl Wasser als auch NH3 enthalten darf.

Diese Trennaufgabe konnte destillativ gelöst werden. Die aus der Verseifung kommenden Brüden werden zunächst in einer Absorptionsstufe in Wasser aufgenommen und diese Lösung dann zwischen Kopf und Sumpf, vorteilhaft in den mittleren bis unteren Bereich einer Destillationskolonne eingespeist. Ist das Keton beispielsweise Aceton, so erfolgt in der Kolonne eine Auftrennung in praktisch aceton- und wasserfreien Ammoniak, der am Kolonnenkopf bei geeignetem Rücklaufverhältnis teilweise oder vollständig kondensiert und kontinuierlich abgezogen wird, sowie in eine 5 - 30 Gew. % Aceton und 0 - 5 Gew. % $NH_3$ enthaltende wäßrige Lösung im Kolonnensumpf, die ebenso kontinuierlich abgezogen wird. Die Kolonne wird bei 100 - 200°C Sumpftemperatur und 1 - 20 barü Druck betrieben. Die Kopftemperatur kann entsprechend zwischen -20 und +55°C liegen.

Vorzugsweise liegt der Druck bei 5 - 18 barü, insbesondere bei 9 - 15 barü; die Sumpftemperatur beträgt entsprechend 140°C - 190°C bzw. 150°C bis 180°C. Das Kopfprodukt kann nach Verdünnen mit Wasser auf die Sollkonzentration von > 50 Gew. % $NH_3$ direkt in die Aminonitrilbildungsstufe zurückgeführt werden. Bei dieser Verfahrensweise konnte auch das eingesetzte Aceton zu über 95 % im Sumpfablauf wiedergefunden werden. Diese Lösung kann direkt oder gewünschtenfalls nach Filtration ggf. über ein Adsorptionsmittel, wie z. B. Aktivkohle, anstelle von frischem Aceton zur Aminonitrilhydrolyse verwendet werden. Hierbei ist es zweckmäßig die Lösung, falls notwendig, zunächst mit Wasser zu verdünnen und ggf. geringe Acetonverluste zu ergänzen bis diejenigen Konzentrationen erreicht sind, die zusammen mit dem Rohaminonitrilgemisch die jeweils gewünschten Sollkonzentrationen an Aceton bzw. Wasser in der Aminonitrilhydrolysestufe ergeben. Die nötigen Hydroxidäquivalente können entweder direkt durch Zumischen zur hier erzeugten wäßrigen Ketonlösung oder auch durch separates Eindosieren einer entsprechenden Alkalihydroxidlösung bzw. Erdalkalihydroxidsuspension der Hydrolysestufe zugeführt werden. Die bei längerer Recyclierung steigenden Nebenproduktanteile, die v. a. aus verschiedenen Pyridinverbindungen bestehen, haben in dem erfindungsgemäßen Verfahren praktisch keinen negativen Einfluß auf die Ausbeute der Aminonitrilhydrolyse. Der Nebenproduktpegel kann jedoch auch durch kontinuierliche Ausschleusung von kleineren Anteilen z. B. von 1 - 10 Gew.% der Sumpflösung aus der Druckdestillation und Ergänzen entsprechender Anteile an Frischlösung auf einem gewünschten Niveau konstant gehalten werden. Die ausgeschleusten Anteile können entweder entsorgt oder wiederaufgearbeitet, insbesondere von Nebenprodukten gereinigt und anschließend wiedereingesetzt werden.

**Gesamtverfahrensbeschreibung**

Das unter den vorstehend beschriebenen Bedingungen durchzuführende Verfahren läßt sich technisch, wie in der Figur gezeigt, in elf Stufen unterteilen, die vorzugsweise vollkontinuierlich aber auch wahlweise teilkontinuierlich oder diskontinuierlich ausgeführt werden können. Im Cyanhydrin-Reaktor 1 wird aus MMP und HCN basenkatalysiert (KAT.) MMP-Cyanhydrin gebildet, das dann im Aminonitril-Reaktor 2, zusammen mit wäßrigem Ammoniak einer Konzentration von > 50 Gew.% aus der Absorptions- und Mischungsstufe 11 umgesetzt wird. Der Cyanhydrin-Reaktor 1 und der Aminonitril-Reaktor 2 können auch zu einer Stufe zusammengefaßt werden. Das gebildete Rohmethioninnitril/Wasser/Ammoniak-Gemisch wird entweder in einer Entspannungsstufe 3 auf einen niedrigeren Druck gebracht und der dabei freigesetzte NH3-Anteil separat abgeführt ( a) oder b) ), oder das Rohgemisch wird unter Verzicht auf die Entspannung direkt in die nachfolgende Hydrolysestufe 4 eingeführt. Dort wird die Aminonitrilhydrolyse mit Hilfe von gleichzeitig

zugeführter wäßriger Alkalihydroxidlösung oder Erdalkalihydroxidsuspension (MOH) und wäßriger Keton-Lösung vorzugsweise in Form des aus der Destillation 10 erhaltenen Sumpfprodukt-Gemisches und ggf. unter Zusatz von frischem Keton (Δ >C=0) bzw. von Wasser (Δ), durchgeführt. Die so gebildete Lösung aus Keton, ggf. $NH_3$ und den oben genannten Reaktionsprodukten in Wasser wird gleichzeitig mit einer entsprechenden wäßrigen Alkalihydroxidlösung oder Erdalkalihydroxidsuspension (MOH) in den Verseifungsreaktor 5 eingebracht und dort umgesetzt. Die dort erzeugte Alkali- oder Erdalkalimethioninatlösung wird nach Passieren einer thermischen Nachbehandlungszone 6, die auch im Verseifungsreaktor 5 integriert sein kann, durch einen Filter 7 gefahren und ggf. anschließend in einer Eindampfstufe 8 weiter konzentriert. Alternativ oder zusätzlich kann eine Eindampfstufe 8a auch nach der Nachbehandlungszone 6 angeordnet sein, der Filter 7 hat dann einen geringeren Flüssigkeitsdurchsatz. Dem Filter wird neues Adsorptionsmittel (C = Aktivkohle und/oder Filterhilfsmittel) und/oder ggf. Filtrationshilfsmittel zugeführt und verbrauchtes entnommen (C-Waste).

Die aus dem Verseifungsreaktor 5 abgeführten Brüden, die Ammoniak, Keton, Wasser und Nebenproduktanteile enthalten, werden, gewünschtenfalls zusammen mit dem aus der Entspannungsstufe 3 stammenden $NH_3$ ( a) ) und den Brüden aus der Eindampfstufe 8 bzw. 8a, in einer Absorptionsstufe 9 in soviel Wasser aufgenommen, daß die erhaltene $NH_3$-Konzentration mindestens 20 Gew.% beträgt. Diese Lösung wird anschließend in die Destillationsanlage 10 geführt. Die dort als Destillationssumpf erhaltene ketonhaltige Lösung geht gewünschtenfalls nach Filtration (7a) über ein Adsorptions- und/oder ein Filterhilfsmittel oder ggf. nach Ausschleusung kleiner Anteile (Waste) in die Hydrolysestufe 4. Der als Kopfprodukt erhaltene $NH_3$ geht, ggf. mit dem aus der Entspannungsstufe 3 erhaltenen $NH_3$-Teilstrom ( b) ), zur Absorptions-und Mischungsstufe 11, in der durch Wasserzugabe die gewünschte $NH_3$-Konzentration eingestellt wird und ggf. geringe $NH_3$-Verluste (Δ-$NH_3$) ergänzt werden.

Die nach diesem Verfahren erzeugte Methioninatlösung kann direkt als Flüssigmethioninformulierung im Futtermittelbereich gemäß DE-OS 31 05 009 oder gewünschtenfalls zur Isolierung von Methionin oder Methioninderivaten nach den literaturbekannten Methoden verwendet werden.

Das hier aufgezeigte Verfahren wird durch die nachfolgenden Beispiele näher erläutert.

Beispiel 1

2-Hydroxy-4-methylmercaptobutyronitril (MMP-Cyanhydrin)

(Pos. 1 in der Figur) In einem 400 l-Kessel mit Ankerrührer, pH-Elektrode, Thermometer, Rückflußkühler, Zudosiervorrichtung und angeschlossenem Blausäureverdampfer wurden 250,0 kg (2400 mol) frisch destillierter 3-(Methylmercapto)-propionaldehyd (MMP) und 110 ml Triethylamin bei 20°C und pH 7,0 vorgelegt. Unter Rühren wurden 66,0 kg (2442 mol) frisch verdampfte, gasförmige Blausäure innerhalb von 7 h so eingeleitet, daß im Kessel eine Temperatur von 30°C nicht überschritten wurde. Durch simultane Zugabe von 130 ml Triethylamin wurde der pH zwischen 5,5 und 7,5 gehalten. Laut HPLC-Analyse war der MMP-Umsatz 100 %. Die MMP-Cyanhydrin-Ausbeute betrug 316 kg mit einem Cyanhydrin-Gehalt von 99,6 Gew.-%. Dieses Cyanhydrin wurde in späteren Beispielen eingesetzt.

Beispiel 2

(Pos.1) In einem 250 l-Kessel mit Ankerrührer, pH-Elektrode, Thermometer, Rückflußkühler, Zudosiervorrichtung und angeschlossenem Vorratsbehälter mit flüssiger HCN wurden unter analogen Bedingungen wie in Beispiel 1 133,9 kg (1285 mol) MMP und 400 ml (= 0,29 kg) Triethylamin mit 35,8 kg (1324 mol) flüssiger Blausäure innerhalb von 4 h umgesetzt. Die MMP-Cyanhydrin-Ausbeute betrug 170,0 kg mit einem Cyanhydrin-Gehalt von 99,2 Gew.-%.

Beispiel 3

2-Amino-4-methylmercaptobuttersäurenitril (D,L-Methioninnitril)

(Pos. 2) In einem 250 ml-Stahlautoklav mit Rührer, Manometer, Innenthermometer, Zuleitung, Pumpe, Vorratsgefäß und Heizbad wurden 51,0 g (2,40 mol) 80 %-iger wäßriger Ammoniak bei 44°C vorgelegt. Anschließend wurden innerhalb von 2 min unter Rühren 51,5 g (0,391 mol) MMP-Cyanhydrin zugepumpt. Die Temperatur stieg dabei auf 57°C, der Druck lag bei 13 barü. Das Reaktionsgemisch wurde noch 20 min bei gleicher Temperatur gerührt, dann im Eisbad abgekühlt und auf Normaldruck entspannt. Eine HPLC-Analyse des Reaktionsgemisches ergab 99,1 % d. Th. Methioninnitril, 0,0 % d. Th. Methioninamid (AM), 0,8 % d. Th. Dinitril (DN) und 0,0 % Dinitrilmonoamid (DNMA).

Beispiel 4

(Pos. 2) Apparatur wie in Beispiel 3. Es wurden 68,0 g (2,40 mol) 60 %-iger wäßriger Ammoniak im Autoklaven

bei 50°C vorgelegt. Anschließend wurden innerhalb von 2 min unter Rühren 53,6 g (0,407 mol) MMP-Cyanhydrin zugepumpt. Die Temperatur stieg dabei auf 58°C, der Druck lag bei 7 barü. Das Reaktionsgemisch wurde noch 10 min bei gleicher Temperatur gerührt, dann im Eisbad abgekühlt und auf Normaldruck entspannt. Eine HPLC-Analyse des Reaktionsgemisches ergab 99,0 % d. Th. Methioninnitril, 0,0 % d. Th. AM, 0,9 % d. Th. Dinitril (DN) und 0,0 % d. Th. DNMA.

Beispiel 5 (Vergleich)

Analog zu Beispiel 3 wurden 102,0 g (2,40 mol) 40 %-iger wäßriger Ammoniak mit 55,2 g (0,419 mol) MMP-Cyanhydrin umgesetzt. Die HPLC-Analyse ergab 98,0 % d. Th. Methioninnitril, 0,3 % d. Th. Methioninamid, 1,4 % d. Th. DN und 0,3 % d. Th. Dinitrilmonoamid (DNMA).

Beispiel 6 (Vergleich)

Analog zu Beispiel 3 wurden 122,4 g (1,80 mol) 25 %-iger wäßriger Ammoniak mit 41,1 g (0,312 mol) MMP-Cyanhydrin umgesetzt. Die HPLC-Analyse ergab 95,8 % d. Th. Methioninnitril, 1,6 % d. Th. Methioninamid, 1,6 % d. Th. DN und 1,0 % d. Th. DNMA.

Beispiel 7 (Vergleich)

(Pos. 2 ohne Pos. 1) In der in Beispiel 3 beschriebenen Apparatur wurden 122,4 g (1,80 mol) 25 %-iger wäßriger Ammoniak im Autoklaven bei 42°C vorgelegt. Anschließend wurden innerhalb von 4 min unter Rühren eine Lösung von 8,5 g (0,314 mol) HCN in 31,7 g (0,304 mol) MMP zugepumpt. Die Temperatur stieg dabei auf 53°C, der Druck lag bei 1 barü. Das Reaktionsgemisch wurde noch 10 min bei gleicher Temperatur gerührt, dann im Eisbad abgekühlt und auf Normaldruck entspannt. Eine HPLC-Analyse des Reaktionsgemisches ergab 91,9 % d. Th. Methioninnitril, 1,8 % d. Th. Methioninamid, 1,7 % d. Th. DN, 1,2 % d. Th. DNMA.

Beispiel 8

(Pos. 2 ohne Pos. 1) Analog Beispiel 7 wurden 51,0 g (1,80 mol) 60 %-iger wäßriger Ammoniak im Autoklaven bei 32°C vorgelegt. Anschließend wurden innerhalb von 0,7 min unter Rühren eine Lösung von 8,5 g (0,314 mol) HCN in 32,2 g (0,309 mol) MMP zugepumpt. Die Temperatur stieg dabei auf 54°C, der Druck lag bei 5 barü. Das Reaktionsgemisch wurde noch 10 min bei gleicher Temperatur gerührt, dann im Eisbad abgekühlt und auf Normaldruck entspannt. Eine HPLC-Analyse des Reaktionsgemisches ergab 97,6 % d. Th. Methioninnitril, 0 % d. Th. Methioninamid, 1,5 Z d. Th. DN, 0,5 % DNMA, 0,2 % d. Th. MMP und 0,2 % d. Th. CH.

Beispiel 9

(Pos. 2) In einem 250 ml-Stahlautoklav mit Rührer, Manometer, Innenthermometer, Zuleitung, Probenentnahmevorrichtung, Pumpe, Vorratsgefäß und Heizbad wurden 68,0 g (2,40 mol) 60 Z-iger wäßriger Ammoniak bei 31°C vorgelegt. Anschließend wurden innerhalb von 2 min unter Rühren 52,9 g (0,402 mol) MMP-Cyanhydrin zugepumpt. Die Temperatur stieg dabei auf 39°C. Das Reaktionsgemisch wurde noch 60 min bei 40°C gerührt und nach 10 bzw. 60 min Proben zur HPLC-Analyse entnommen. Dabei ergaben sich folgende Ausbeutewerte,

| | Methioninnitril | MMP | MMP-CH | DN |
|---|---|---|---|---|
| nach 10 min: | 63,5 | 2,4 | 34,0 | 0,1 % d. Th. |
| nach 60 min: | 97,4 | 0,6 | 0,4 | 1,6 % d. Th. |

Beispiel 10 (Vergleich)

(Pos. 2) Analog zu Beispiel 3 wurden 116,3 g (2,60 mol) 38 %-iger wäßriger Ammoniak im Autoklaven bei 68°C vorgelegt. Anschließend wurden innerhalb von 1,6 min unter Rühren 54,3 g (0,412 mol) MMP-Cyanhydrin zugepumpt. Die Temperatur stieg dabei auf 80°C, der Druck lag bei 5 barü. Das Reaktionsgemisch wurde noch 10 min bei gleicher Temperatur gerührt, dann im Eisbad abgekühlt und auf Normaldruck entspannt. Eine HPLC-Analyse des Reaktionsgemisches ergab 93,0 % d. Th. Methioninnitril, 0,45 % d. Th. Methioninamid, 1,35 % d. Th. MMP, 4,60 % d. Th. DN, 0,55 % d. Th. DNMA.

Beispiel 11 (Vergleich)

(Pos. 2) Analog Beispiel 3 wurden 68,0 g (2,40 mol) 60 %-iger wäßriger Ammoniak und 4,7 g (0.08 mol) Aceton im Autoklaven bei 44°C vorgelegt. Anschließend wurden innerhalb von 2 min unter Rühren 53,7 g (0,408 mol) MMP-Cyanhydrin zugepumpt. Die Temperatur stieg dabei auf 56°C, der Druck lag bei 6 barü. Das Reaktionsgemisch wurde noch 10 min bei gleicher Temperatur gerührt, dann im Eisbad abgekühlt und auf Normaldruck entspannt. Eine HPLC-Analyse des Reaktionsgemisches ergab 91,6 % d. Th. Methioninnitril und 8,4 % d. Th. MMP.

Beispiel 12

(Pos. 2) Zur kontinuierlichen Verfahrensweise wurden in ein beheizbares Reaktionsrohr mit vorgeschalteter Mischstrecke, Innentemperaturmessung, Probenentnahmevorrichtung und angeschlossener MMP-CH-Zuleitung mit Vorratsgefäß und $NH_3$-Zuleitung mit Vorratsgefäß und zwischengeschaltetem Wärmetauscher gleichzeitig pro Stunde 2,55 kg (89,8 mol) auf 40°C vorgeheizter, wäßriger 60 %-iger Ammoniak und 1,923 kg (14,60 mol) MMP-Cyanhydrin eindosiert. Die eingestellte Innentemperatur betrug am Rohreingang 55°C, am Rohrausgang 56°C, der Druck betrug 7,8 barü, die Verweilzeit 17,3 min. Die HPLC-Analyse von am Rohrausgang entnommenen Proben ergab 97,2 % d. Th. Methioninnitril, 0,3 Z d. Th. Methioninamid, 0,5 % d. Th. MMP und 1,8 % d. Th. DN.

Beispiel 13

(Pos.2) Analog zu Beispiel 12 wurden pro Stunde 2,45 kg (86,3 mol) 60 %-iger wäßriger Ammoniak mit 2,371 kg (18,0 mol) MMP-Cyanhydrin bei einer Verweilzeit von 21,8 min umgesetzt. Die HPLC-Analyse ergab 95,2 Z d. Th. Methioninnitril, 0,0 % d. Th. Methioninamid, 1,7 % d. Th. DN, 0,7 % d. Th. MMP-CH, 0,4 % d. Th. MMP.

Beispiel 14

(Pos. 2) Analog Beispiel 12 wurden gleichzeitig pro Stunde 2,07 kg (97,2 mol) auf 53°C vorgeheizter, wäßriger 80 %-iger Ammoniak und 1,917 kg (14,56 mol) MMP-Cyanhydrin eindosiert. Die eingestellte Innentemperatur betrug am Rohreingang 57°C, am Rohrausgang 56°C, der Druck betrug 14,4 barü, die Verweilzeit 24,5 min. Die HPLC-Analyse von am Rohrausgang entnommenen Proben ergab 98,2 % d. Th. Methioninnitril, 0,0 % d. Th. Methioninamid, 1,3 % d. Th. DN und 0,4 % d. Th. MMP.

Beispiel 15

D,L-Methioninamidlösung

(Pos. 2, 3 und 4) In einem 250 ml Stahlautoklav mit Rührer, Manometer, Innenthermometer, Zuleitung, Pumpe, Vorratsgefäß und Heizbad wurden 50,0 g (1,76 mol) 60 %-iger wäßriger Ammoniak bei 45°C vorgelegt. Anschließend wurden innerhalb von 1,5 min unter Rühren 37,25 g (0,283 mol) MMP-Cyanhydrin zugepumpt. Die Temperatur stieg dabei auf 54°C, der Druck lag bei 7 barü. Das Reaktionsgemisch wurde noch 10 min bei gleicher Temperatur gerührt und dann unter gleichzeitiger Kühlung auf Normaldruck entspannt.

In das 15°C warme Gemisch wurde unter gleichzeitiger Wasserkühlung eine Lösung von 2,5 g (0,063 mol) NaOH und 8,8 g (0,152 mol) Aceton in 110 g (6,11 mol) Wasser eingepumpt, wobei die Temperatur auf 27°C stieg. Das Reaktionsgemisch wurde noch 30 min bei 25°C gerührt und dann sofort analysiert. Die klare, blaßgelbe Lösung (201,1 g) hatte folgende Zusammensetzung:

| | |
|---|---|
| 19,93 Gew.-% Methioninamid, | 95,60 % d. Th. |
| 0,38 Gew.-% Methionin, | 1,80 % d. Th. |
| 0,06 Gew.-% IM1, | 0,35 % d. Th. |
| 0,46 Gew.-% IM2, | 1,70 % d. Th. |
| 0,05 Gew.-% MMP, | 0,35 % d. Th. |

Beispiel 16

(Pos. 2, 3 und 4) Analog Beispiel 15 wurden 51,0 g (1,80 mol) 60 %-iger wäßriger Ammoniak im Autoklaven bei 45°C vorgelegt. Anschließend wurden innerhalb von 1,5 min unter Rühren 42,0 g (0,319 mol) MMP-Cyanhydrin zugepumpt. Die Temperatur stieg dabei auf 54°C, der Druck lag bei 7 barü. Das Reaktionsgemisch wurde noch 10 min bei

gleicher Temperatur gerührt und dann unter gleichzeitiger Kühlung auf Normaldruck entspannt.

In das 15°C warme Gemisch wurde unter gleichzeitiger Wasserkühlung eine Lösung von 12,2 g (0,31 mol) NaOH und 8,8 g (0,152 mol) Aceton in 113 g (6,27 mol) Wasser eingepumpt, wobei die Temperatur auf 27°C stieg. Das Reaktionsgemisch wurde noch 30 min bei 25°C gerührt und dann sofort analysiert. Die trübe, blaßgelbe Lösung (208,3 g) hatte folgende Zusammensetzung:

| | |
|---|---|
| 18,8 Gew.-% Methioninamid, | 82,8 % d. Th. |
| 2,8 Gew.-% Methionin, | 12,2 % d. Th. |
| 0,0 Gew.-% IM1, | 0 % d. Th. |
| 0,77 Gew.-% IM2, | 2,7 % d. Th. |
| 0,17 Gew.-% MMP, | 1,1 % d. Th. |

Beispiel 17

An ein beheizbares Strömungsrohr mit Innentemperaturmessung (Pos. 2) ist über eine vorgeschaltete Mischstrecke eine MMP-CH-Zuleitung, die mit einem Vorratsgefäß verbunden ist, und eine Ammoniak-Zuleitung angeschlossen, die über einen zwischengeschalteten Wärmetauscher mit einer Absorptions- und Mischungsstufe (Pos. 11) verbunden ist. Das Strömungsrohr ist über ein automatisches Druckhalteventil mit einem gerührten Entspannungsgefäß (Pos. 3) verbunden, dessen Gasraum über eine Leitung mit einer Absorptionsstufe (Pos. 9) verbunden ist, und dessen Bodenablauf über eine Pumpe zur Hydrolysestufe (Pos. 4) führt. Die Hydrolysestufe ist thermostatisierbar und besteht aus einem Schlaufenreaktor mit Umwälzpumpe und nachgeschaltetem Strömungsrohr. Der Schlaufenreaktor ist eingangsseitig noch über Förderleitung und Pumpe mit einem Vorratsgefäß für wäßrige Aceton/NaOH-Lösung verknüpft und das Strömungsrohr ist über eine Druckregelung mit einem Auffanggefäß verbunden. Die Absorptionsstufe (Pos. 9) ist ein $NH_3$-Absorptionssystem, das sich aus einem Sumpfbehälter mit Umwälzpumpe und Quencher und einer aufgesetzten Glockenbodenkolonne zusammensetzt.

Der Kolonnenkopf wird über Förderleitung und Pumpe aus einem Frischwasserbehälter versorgt. Der Sumpfablauf mündet in ein Auffanggefäß.

In das Strömungsrohr (Pos. 2) wurden jeweils pro Stunde durchschnittlich 1,925 kg (14,61 mol) MMP-Cyanhydrin sowie 2,55 kg (89,8 mol) auf 40°C vorgeheizter 60 %-iger Ammoniak aus Pos. 11 jeweils flußgeregelt eindosiert. Die eingestellte Innentemperatur betrug 55°C am Rohreingang und 56°C am Rohrausgang, der Druck 7,8 barü, die Verweilzeit 23 min. Der Druck in dem Entspannungsgefäß (Pos. 3) lag bei 1,2 barü, die Temperatur bei 35°C. Der Rohaminonitrilpegel im Entspannungsgefäß wurde über eine konstante Fördermenge zur Hydrolysestufe (Pos. 4) konstant gehalten.

In den Schlaufenreaktor der Hydrolysestufe (Pos. 4) wurden neben der Rohaminonitrilmischung aus der Entspannungsstufe (Pos. 3) pro Stunde 6,40 kg einer wäßrigen Lösung mit 7,25 Gew.-% (0,464 kg, 7,99 mol) recycliertem Aceton, 2,0 Gew.-% (0,128 kg, 3,2 mol) NaOH und 3,3 Gew.-% $NH_3$ (0,211 kg, 12,4 mol) aus einem Vorratsbehälter eingepumpt. Die Temperatur in der Schlaufe betrug 27°C, im Reaktionsrohr 26°C, die gesamte Verweilzeit 50 min.

Die Temperatur in der $NH_3$-Absorption (Pos. 9) wurde bei 22°C und der Druck bei 1,2 barü gehalten. Stündlich wurden im Ablauf der Hydrolysestufe (Pos. 4) im Mittel 10,58 kg einer blaßgelb gefärbten wäßrigen Produktlösung mit folgender Zusammensetzung erhalten:

| | |
|---|---|
| 19,19 Gew.-% Methioninamid, | 93,8 % d. Th. |
| 0,31 Gew.-% Methionin, | 1,5 % d. Th. |
| 0,38 Gew.-% IM2, | 1,5 % d. Th. |
| 0,26 Gew.-% MMP, | 1,8 % d. Th. |

Beispiel 18

D,L-Natriummethioninatlösung

(Pos. 2, 3, 4, 5 und 6) An einen 250 ml-Stahlautoklav mit Rührer, Manometer. Innenthermometer und Heizbad sind eine Zuleitung mit Pumpe und Vorratsgefäß angeschlossen.

Es wurden 38,3 g (1,80 mol) 80 %-iger wäßriger Ammoniak im Autoklaven bei 42°C vorgelegt und anschließend unter Rühren 37,80 g (0,287 mol) MMP-Cyanhydrin innerhalb von 1,5 min zugepumpt. Das Reaktionsgemisch wurde noch 20 min bei 56°C gerührt und unter gleichzeitigem Kühlen von 11 barü Druck auf Normaldruck entspannt. Dabei wurden 14,4 g (0,84 mol) $NH_3$ zurückgewonnen. In das 20°C warme Gemisch wurde unter gleichzeitiger Wasserkühlung eine Lösung von 2,5 g (0,063 mol) NaOH und 8,8 g (0,152 mol) Aceton in 122 g (6,77 mol) Wasser eingepumpt, wobei die Innentemperatur auf 25°C stieg. Bei gleicher Temperatur wurde noch 35 min gerührt. Dann wurden 18,6 g (0,233 mol) 50 %-ige Natronlauge zugepumpt und bei Normaldruck 30 min lang bei 100 - 105°C gerührt. Anschließend wurde noch 30 min bei 160°C und 15 barü gerührt. Die erhaltene hellgelbe Lösung wurde filtriert und bei Normaldruck eingeengt. Es wurden 102,3 g einer gelben Lösung mit folgender Zusammensetzung erhalten:

| | |
|---|---|
| 40,15 Gew.-% Methionin, | 95,9 % d. Th. |
| Spur DS1 | |
| 0,42 Gew.-% DS2, | 0,64 % d. Th. |
| < 10 ppm Cyanid. | |

Beispiel 19

Analog Beispiel 18 wurden 51,0 g (1,80 mol) 60 %-iger wäßriger Ammoniak im Autoklaven bei 44°C vorgelegt und anschließend unter Rühren 38,5 g (0,292 mol) MMP-Cyanhydrin innerhalb von 1,5 min zugepumpt. Das Reaktionsgemisch wurde noch 11 min bei 55°C gerührt und unter gleichzeitigem Kühlen von 6 barü Druck auf Normaldruck entspannt. Dabei wurden 12,3 g $NH_3$ zurückgewonnen. In das 20°C warme Gemisch wurde unter gleichzeitiger Wasserkühlung eine Lösung von 2,5 g (0,063 mol) NaOH und 8,8 g (0,152 mol) Aceton in 115 g (6,38 mol) Wasser eingepumpt, wobei die Innentemperatur auf 25°C stieg. Bei gleicher Temperatur wurde noch 32 min gerührt. Dann wurden 18,6 g (0,233 mol) 50 %-ige Natronlauge zugepumpt und noch 45 min bei 160°C und 21 barü gerührt. Die erhaltene hellgelbe Lösung wurde filtriert und bei Normaldruck eingeengt. Nach Abkühlung auf Raumtemperatur wurden 100,8 g einer gelben Lösung mit folgender Zusammensetzung erhalten:

| | |
|---|---|
| 41,7 Gew.-% Methionin, | 96,5 % d. Th. |
| < 0,04 Gew.-% DS1, | < 0,01 % d. Th. |
| 0,42 Gew.-% DS2, | 0,6 % d. Th. |
| < 10 ppm Cyanid. | |

Beispiel 20

Natriummethionatlösung wurde analog zu Beispiel 19 hergestellt aus 50,0 g (1,76 mol) 60 %-iger Ammoniak, 39,3 g (0,298 mol) MMP-Cyanhydrin, einer Lösung von 2,5 g (0,063 mol) NaOH und 3,6 g (0,062 mol) Aceton in 79,6 g (4,42 mol) Wasser und 18,8 g (0,235 mol) 50 %-iger Natronlauge. Erhalten wurden 101,8 g hellbraune Lösung mit folgender Zusammensetzung:

| | |
|---|---|
| 41,2 Gew.-% Methionin, | 94,3 % d. Th. |
| 0,20 Gew.-% DS1, | 0,5 % d. Th. |
| 0,66 Gew.-% DS2, | 1,0 % d. Th. |

Beispiel 21 (Vergleich)

Analog Beispiel 18 wurden 122,2 g (1,80 mol) 25 %-iger wäßriger Ammoniak im Autoklaven bei 50°C vorgelegt und anschließend unter Rühren 39,4 g (0,299 mol) MMP-Cyanhydrin innerhalb von 1,5 min zugepumpt. Das Reaktionsgemisch wurde noch 10 min bei 55°C und 2 barü gerührt und anschließend abgekühlt. In das 20°C warme drucklose Gemisch wurden 6,2 g (0,062 mol) 40 %-ige Natronlauge und 3,6 g (0,062 mol) Aceton eingepumpt, wobei die Innentemperatur auf 36°C stieg. Anschließend wurde die Mischung noch 60 min bei 33°C gerührt. Dann wurden 19,1 g (0,239 mol) 50 %-ige Natronlauge zugepumpt und noch 45 min bei 160°C und 21 barü gerührt. Die erhaltene Lösung wurde filtriert und bei Normaldruck eingeengt. Nach Abkühlung auf Raumtemperatur wurden 104,2 g einer rötlichbraunen Lösung mit folgender Zusammensetzung erhalten:

| | |
|---|---|
| 39,65 Gew.-% Methionin, | 92,6 % d. Th. |
| 0,59 Gew.-% DS1, | 1,5 % d. Th. |
| 0,56 Gew.-% DS2, | 0,8 % d. Th. |

Beispiel 22

An ein beheizbares Strömungsrohr mit Innentemperaturmessung (Pos. 2) ist über eine vorgeschaltete Mischstrecke eine MMP-CH-Zuleitung, die mit einem Vorratsgefäß verbunden ist, und eine Ammoniak-Zuleitung angeschlossen, die über einen zwischengeschalteten Wärmetauscher mit einer Absorptions- und Mischungsstufe (Pos. 11) verbunden ist. Das Strömungsrohr (Pos. 2) ist direkt mit einer Hydrolysestufe (Pos. 4) verbunden, die thermostatisierbar ist und aus einem Schlaufenreaktor mit Umwälzpumpe und nachgeschaltetem Strömungsrohr besteht. Der Schlaufenreaktor ist eingangsseitig noch über Förderleitung und Pumpe mit einem Vorratsgefäß für wäßrige Aceton/NaOH-Lösung verknüpft und ausgangsseitig über das Strömungsrohr und eine Druckregelung mit einem Verseifungsreaktor (Pos. 5) verbunden. Der Verseifungsreaktor besteht aus einer Glockenbodenkolonne, deren oberster Boden über Einspeiseleitung und Pumpe mit einem Natronlaugevorratsbehälter und deren Kopf über eine Druckhaltung mit einer Absorptionsstufe (Pos. 9) verbunden ist. Der Sumpfteil besteht aus einem Umlaufverdampfer. Der niveaugeregelte Sumpfablauf des Verseifungsreaktors ist über einen Zwischenbehälter und eine Pumpe mit einer Nachbehandlungszone (Pos. 6) verknüpft. Die Nachbehandlungszone besteht aus einem Vorheizer und einer nachgeschalteten Verweilzeitstrecke und ist über eine thermostatisierbare Leitung und eine Druckhaltung direkt mit einem Filter (Pos. 7) verbunden. Der Filter besteht aus einem Feinfilter, dessen Ablauf in eine Eindampfstufe (Pos. 8) führt. Die Eindampfstufe ist ein Eindampfsystem, bestehend aus Fallfilmverdampfer, nachgeschaltetem Kondensator und Produktauffanggefäß. Die Absorptionsstufe (Pos. 9) dient als NH3-Absorptionssystem, das sich aus einem Sumpfbehälter mit Umwälzpumpe und Quencher und einer aufgesetzten Glockenbodenkolonne zusammensetzt. Der Kolonnenkopf wird über Förderleitung und Pumpe aus einem Frischwasserbehälter versorgt, während in den Quencher die Brüdenleitung vom Kopf der Verseifungskolonne (Pos. 5) und die Kondensatleitung der Eindampfstufe (Pos. 8) einmünden. Der Sumpfablauf der Absorptionsstufe (Pos. 9) ist über eine Pumpe mit einer Destillationsanlage (Pos. 10) verbunden, die als Druckdestillationssystem zur kontinuierlichen Abtrennung von $NH_3$ aus dem Einspeisegemisch dient und als Glockenbodenkolonne ausgeführt ist. Die Einspeisung aus der Absorptionsstufe (Pos. 9) erfolgt in Kolonnenmitte.

Der Sumpfteil besteht aus einem Umlaufverdampfer, der einen niveaugeregelten, kühlbaren Ablauf besitzt und über diesen mit einem Auffanggefäß gekoppelt ist, an das sich wiederum der Vorratsbehälter für die wäßrige Aceton/NaOH-Lösung anschließt.

Der Kopfteil besteht aus einem Kondensator mit anschließendem Auffanggefäß, das über eine Flußregelung mit der Absorptions- und Mischungsstufe (Pos. 11) verbunden ist, die aus einem druckstabilen Rührkessel besteht, der zusätzlich mit einer Flüssigammoniak- und mit einer Frischwasserzufuhrmöglichkeit versehen ist.

In das Strömungsrohr (Pos. 2) wurden jeweils pro Stunde durchschnittlich 1,774 kg (13,47 mol) MMP-Cyanhydrin sowie 2,55 kg (89,8 mol) auf 40°C vorgeheizter 60 %-iger Ammoniak aus Pos. 11 jeweils flußgeregelt eindosiert. Die eingestellte Innentemperatur betrug 55°C am Rohreingang und 50°C am Rohrausgang, der Druck 7,8 barü, die Verweilzeit 23 min. In den Schlaufenreaktor (Pos. 4) wurden pro Stunde 6,0 kg einer wäßrigen Lösung mit 7,25 Gew.-% (7,50 mol) Aceton und 2,0 Gew.-% (3,00 mol) NaOH aus einem Vorratsbehälter eingepumpt. Die Temperatur in der Schlaufe betrug 29°C im Reaktionsrohr 25°C, die gesamte Verweilzeit 50 min. In dem Verseifungsreaktor (Pos. 5) wurden jeweils pro Stunde 0,86 kg (10,75 mol) 50 %-ige Natronlauge zugepumpt. Die Temperatur betrug 132°C im Sumpf und 118°C am Kopf der Kolonne, der Druck 1,7 barü, die gesamte Verweilzeit 26 min.

Durch Kühlen und Zupumpen von durchschnittlich 1 l Wasser pro Stunde in die Brüdenabsorption (Pos. 9) wurde dort die Temperatur auf 19 - 22°C und der Druck auf 0,3 - 1,0 barü gehalten. Hieraus wurden pro Stunde 5,5 - 6,5 1 Lösung in die Druckdestillationskolonne eingespeist. Die Temperatur betrug 161 - 163°C im Sumpf und 28 - 29°C am Kopf, der Druck 10 barü, das eingestellte Rücklaufverhältnis 0,5 - 0,6. Im Auffanggefäß des Sumpfablaufs wurden pro Stunde im Durchschnitt 4,0 kg einer 11 - 12 Gew-% Aceton und 2,0 - 2,7 Gew.-% $NH_3$ enthaltenden, wäßrigen Lösung gesammelt. Diese wurde mit solchen Mengen Wasser, Aceton und Natronlauge versetzt, bis die erhaltene Gesamtkonzentration der oben genannten Sollkonzentration entsprach und anschließend in den Vorratsbehälter für die Hydrolysestufe (Pos. 4) gefüllt. Aus dem Auffanggefäß des Kopfteils der Destillationskolonne wurden pro Stunde 2,5 l kondensierter Ammoniak in Pos. 11 gefahren zusammen mit 1 l Frischwasser pro Stunde. Von Zeit zu Zeit wurden geringfügige Verluste durch entsprechende Ergänzung mit Frisch-$NH_3$ und Frischwasser ersetzt. Die Temperatur im Nachreaktor (Pos. 6) betrug 180°C, der Druck 12 barü und die Verweilzeit 20 min. Nach Entspannung auf Normaldruck und Abkühlung auf 90 - 95°C wurde die Lösung filtriert (Pos. 7) und anschließend im Fallfilmverdampfer (Pos. 8) bei 135 - 140°C/0,5 bar konzentriert. Stündlich wurden im Ablauf der Eindampfstufe (Pos. 8) im Mittel 4,69 kg einer gelb gefärbten wäßrigen Natriummethioninatlösung mit folgender Zusammensetzung erhalten:

| | |
|---|---|
| 40,7 Gew.-% Methionin, | 95,0 % d. Th. |
| 0,15 Gew.-% DS1, | 0,4 % d. Th. |
| 0,18 Gew.-% DS2, | 0,3 % d. Th. |
| 6,74 Gew.-% Natrium | |

(fortgesetzt)

| | |
|---|---|
| < 5 ppm Cyanid | |

Beispiel 23

Es wird wie in Beispiel 22 verfahren, wobei zwischen das Strömungsrohr (Pos. 2) und die Hydrolysestufe (Pos. 4) eine Entspannungsstufe (Pos. 3) zwischengeschaltet ist.

Die Entspannungsstufe (Pos. 3) ist ein gerührtes Entspannungsgefäß, dessen Gasraum mit der Absorptionsstufe (Pos. 9) und dessen Bodenablauf über eine Pumpe mit der Hydrolysestufe (Pos. 4) verbunden ist.

Der Filter (Pos. 7) besteht aus zwei in Reihe geschalteten Aktivkohlefiltersäulen, die von unten nach oben durchströmt werden, so daß der Ablauf der zweiten Säule in die Eindampfstufe (Pos. 8) führt.

In das Strömungsrohr (Pos. 2) wurden jeweils pro Stunde durchschnittlich 1,931 kg (14,66 mol) MMP-Cyanhydrin sowie 2,61 kg (92,0 mol) auf 40°C vorgeheizter 60 %-iger Ammoniak (aus Pos. 11) jeweils flußgeregelt eindosiert. Die eingestellte Innentemperatur betrug 55°C am Rohreingang und 56°C am Rohrausgang, der Druck 7,8 barü, die Verweilzeit 23 min. Das Reaktionsgemisch wurde in der Entspannungsstufe Pos. 3 auf 0,9 - 1,2 barü bei 30°C entspannt und in den Schlaufenreaktor (Pos. 4) gepumpt, in den gleichzeitig pro Stunde 6,0 kg einer wäßrigen Lösung mit 7,25 Gew.-Z (7,5 mol) Aceton und 2,0 Gew.-% (3,0 mol) NaOH aus einem Vorratsbehälter eingepumpt wurden. Die Temperatur in der Schlaufe betrug 26°C, im Reaktionsrohr 25°C, die gesamte Verweilzeit 50 min.

Im Verseifungsreaktor (Pos. 5) wurden jeweils pro Stunde 0,92 kg (11,50 mol) 50 %-ige Natronlauge zugepumpt. Die Temperatur betrug 135°C im Sumpf und 122°C am Kopf der Kolonne, der Druck 1,7 barü, die gesamte Verweilzeit 26 min.

Durch Kühlen und Zupumpen von durchschnittlich 1,8 l Wasser in die Brüdenabsorption (Pos. 9) wurde dort die Temperatur auf 21 - 27°C und der Druck auf 0,9 - 1,2 barü gehalten. Aus Pos. 9 wurden pro Stunde 5,5 - 6,5 l Lösung in die Druckdestillationskolonne eingespeist. Die Temperatur betrug 160 - 161°C im Sumpf und 28 - 29°C am Kopf, der Druck 10 barü, das eingestellte Rücklaufverhältnis 0,5 - 0,6. Im Auffanggefäß des Sumpfablaufs wurden pro Stunde durchschnittlich 4,0 kg einer 10 - 12 Gew.-% Aceton und 2,0 - 3,0 Gew.-% $NH_3$ enthaltenden, wäßrigen Lösung gesammelt. Diese wurde mit solchen Mengen Wasser, Aceton und Natronlauge versetzt, bis die erhaltene Gesamtkonzentration der oben genannten Sollkonzentration entsprach und anschließend in den Vorratsbehälter für die Hydrolysestufe (Pos. 4) gefüllt. Aus dem Auffanggefäß des Kopfteils der Destillationskolonne wurden pro Stunde 2,5 l kondensierter Ammoniak in Pos. 11 gefahren zusammen mit 1 l Frischwasser pro Stunde. Von Zeit zu Zeit wurden geringfügige Verluste durch entsprechende Ergänzung mit Frisch-$NH_3$ und Frischwasser ersetzt. Die Temperatur im Nachreaktor (Pos. 6) betrug 180°C, der Druck 12 barü und die Verweilzeit 20 min. Nach Entspannung auf Normaldruck und Abkühlung auf 80°C wurde die Lösung bei dieser Temperatur über insgesamt 13 kg A-Kohle (Pos. 7) gefahren und anschließend im Fallfilmverdampfer (Pos. 8) bei 135 - 140°C/0,5 bar konzentriert. Stündlich wurden im Ablauf von Pos. 8 im Mittel 4,945 kg einer hellgelb gefärbten, wäßrigen Natriummethioninatlösung mit folgender Zusammensetzung erhalten:

| | |
|---|---|
| 42,16 Gew.-% Methionin, | 95,3 % d. Th. |
| 0,18 Gew.-% DS1, | 0,4 % d. Th. |
| 0,20 Gew.-% DS2, | 0,3 % d. Th. |
| 6,74 Gew.-% Natrium | |
| < 2 ppm Cyanid | |

Beispiel 24

Aufbau wie in Beispiel 23 beschrieben. In das Strömungsrohr (Pos. 2) wurden jeweils pro Stunde durchschnittlich 1,978 kg (15,02 mol) MMP-Cyanhydrin sowie 2,01 kg (94,4 mol) auf 40°C vorgeheizter 80 %-iger Ammoniak (aus Pos. 11) jeweils flußgeregelt eindosiert. Die eingestellte Innentemperatur betrug 58°C am Rohreingang und 56°C am Rohrausgang, der Druck 14,2 barü, die Verweilzeit 24,6 min. Das Reaktionsgemisch wurde in der Entspannungsstufe (Pos. 3) auf 0,9 - 1,0 barü bei 30°C entspannt und in den Schlaufenreaktor (Pos. 4) gepumpt, in den gleichzeitig pro Stunde 6,7 kg einer wäßrigen Lösung mit 6,55 Gew.-% (7,56 mol) Aceton und 1,8 Gew.-% (3,02 mol) NaOH aus einem Vorratsbehälter eingepumpt wurden. Die Temperatur in der Schlaufe betrug 26°C, im Reaktionsrohr 25°C, die gesamte Verweilzeit 50 min.

Im Verseifungsreaktor (Pos. 5) wurden jeweils pro Stunde 0,96 kg (11,98 mol) 50 %-ige Natronlauge zugepumpt. Die Temperatur betrug 133°C im Sumpf und 118°C am Kopf der Kolonne, der Druck 1,7 barü, die gesamte Verweilzeit 26 min.

Durch Kühlen und Zupumpen von durchschnittlich 2,4 1 Wasser pro Stunde in die Brüdenabsorption (Pos. 9) wurde dort die Temperatur auf 19 - 22°C und der Druck auf 0,9 - 1,0 barü gehalten. Aus Pos. 9 wurden pro Stunde 5,5 - 6,5 l Lösung in die Druckdestillationskolonne eingespeist. Die Temperatur betrug 159 - 161°C im Sumpf und 28 - 29°C am Kopf, der Druck 10 barü, das eingestellte Rücklaufverhältnis 0,4 - 0,5. Im Auffanggefäß des Sumpfablaufs wurden pro Stunde durchschnittlich 4,0 kg einer 10 - 12 Gew.-% Aceton und 2,0 - 3,0 Gew.-% $NH_3$ enthaltenden, wäßrigen Lösung gesammelt. Diese wurde mit solchen Mengen Wasser, Aceton und Natronlauge versetzt, bis die erhaltene Gesamtkonzentration der oben genannten Sollkonzentration entsprach und anschließend in den Vorratsbehälter für die Hydrolysestufe (Pos. 4) gefüllt. Aus dem Auffanggefäß des Kopfteils der Destillationskolonne wurden pro Stunde 2,5 1 kondensierter Ammoniak in Pos. 11 gefahren zusammen mit 0,4 l Frischwasser pro Stunde. Von Zeit zu Zeit wurden geringfügige Verluste durch entsprechende Ergänzung mit Frisch-$NH_3$ und Frischwasser ersetzt. Die Temperatur im Nachreaktor (Pos. 6) betrug 180°C, der Druck 12 barü und die Verweilzeit 20 min. Nach Entspannung auf Normaldruck und Abkühlung auf 80°C wurde die Lösung bei dieser Temperatur über insgesamt 13 kg A-Kohle (Pos. 7) gefahren und anschließend im Fallfilmverdampfer (Pos. 8) bei 135 - 140°C/0,5 bar konzentriert. Stündlich wurden im Ablauf von Pos. 8 im Mittel 4,87 kg einer hellgelb gefärbten, wäßrigen Natriummethioninatlösung mit folgender Zusammensetzung erhalten:

| | |
|---|---|
| 44,25 Gew.-% Methionin, | 96,2 % d. Th. |
| 0,14 Gew.-% DS1, | 0,3 % d. Th. |
| 0,18 Gew.-% DS2, | 0,2 % d. Th. |
| 7,08 Gew.-% Natrium | |
| < 5 ppm Cyanid | |

Beispiel 25

Calciummethioninatlösung

(Pos. 5) In einem 350 ml Stahlautoklav mit Rührer, Monometer, Innenthermometer und Heizbad wurde eine Lösung von 50,0 g (0,330 mol) Methioninamid (97,8 %ig) in 223,2 g Wasser unter Rühren mit 13,1 g (0,170 mol) $Ca(OH)_2$ (96 %ig) versetzt und nach Aufheizen auf 130°C bei dieser Temperatur und 2 barü Druck 120 min lang gerührt. Die Reaktionsmischung wurde unter gleichzeitigem Kühlen auf Normaldruck entspannt. Erhalten wurden 286,2 g gelbliches Filtrat mit einem Gehalt von 17,06 Gew.-% Methionin, 99,2 % d. Th. und 0,08 Gew.-% Methioninamid, 0,5 % d. Th..

## Patentansprüche

1. Verfahren zur Herstellung von Methionin oder einem seiner Salze durch Hydrolyse von Methioninnitril zum Methioninamid in Gegenwart eines Ketons und anschließender Verseifung des Amids mit einer Base,
   **dadurch gekennzeichnet**,
   daß während und/oder nach der Verseifung des Amids Ammoniak, Keton und Wasser gemeinsam bei einer Temperatur ≥ 85°C und/oder unter Anlegen eines Vakuums abgezogen werden.

2. Verfahren nach Anspruch 1,
   **dadurch gekennzeichnet**,
   daß das Methioninnitril durch Umsetzung von Methylmercaptopropionaldehyd (MMP) mit Blausäure und Ammoniak oder durch Umsetzung des entsprechenden Cyanhydrins (CH) mit Ammoniak oder durch Umsetzung eines beliebigen Gemisches aus MMP/Blausäure und dem entsprechenden Cyanhydrin mit Ammoniak erhalten wird.

3. Verfahren nach Anspruch 1 oder 2,
   **dadurch gekennzeichnet**,
   daß das Keton vom Ammoniak abgetrennt wird.

4. Verfahren nach Anspruch 3,
   **dadurch gekennzeichnet**,
   daß der im wesentlichen ketonfreie Ammoniak der Nitrilsynthese gemäß Anspruch 2 zugeführt wird.

5. Verfahren zur Herstellung von Methioninamid, Methionin oder von einem seiner Salze durch Umsetzung von Methylmercaptopropionaldehyd (MMP) mit Blausäure und Ammoniak oder des entsprechenden Cyanhydrins (CH)

mit Ammoniak oder einem Gemisch aus MMP, CH, Blausäure und Ammoniak zum Methioninnitril, anschließender Hydrolyse des Nitrils in Gegenwart eines Ketons zum Amid, ggf. anschließender Verseifung des Amids, Abtrennung des Ammoniaks und Zurückführen desselben zumindest teilweise zu der Nitrilsynthese,
**dadurch gekennzeichnet,**
daß der Ammoniak vor der Zurückführung im wesentlichen vom Keton befreit wird.

**6.** Verfahren nach Anspruch 5,
**dadurch gekennzeichnet**,
daß der der Nitrilsynthese zugeführte recyclierte Ammoniak weniger als 25 meq bezüglich des Cyanhydrins und/oder Aldehyds enthält.

**7.** Verfahren zur Herstellung von Methionin oder Methioninaten durch Hydrolyse von Methioninnitril bzw. Methioninamid in Gegenwart einer Base,
**dadurch gekennzeichnet**,
daß die Anfangskonzentration des Amids ≤ 25 Gew.-% gehalten wird, sofern die Verseifungstemperatur bei 160 °C oder höher liegt.

**8.** Verfahren nach Anspruch 7,
**dadurch gekennzeichnet**,
daß die Verseifungstemperatur nicht über 200 °C liegt.

**9.** Verfahren nach Anspruch 1,
**dadurch gekennzeichnet,**
daß zur Abtrennung von Ammoniak aus einem gemeinsam abgezogenen Ammoniak-Keton-Wasser-Gemisch das Gemisch zwischen Kopf und Sumpf in eine Kolonne eingespeist wird, daß die Kolonne bei einem Druck zwischen 1 - 20 barü gehalten wird, daß die Sumpftemperatur zwischen 100 und 200 °C gehalten wird, und daß das Keton, Wasser und ggf. geringe Ammoniakmengen aus dem Sumpf abgezogen werden.

**10.** Verfahren nach Anspruch 9,
**dadurch gekennzeichnet,**
daß bei einem Druck zwischen 5 und 18 barü, insbesondere 9 bis 15 barü und eine Temperatur von 140 bis 190 °C, insbesondere 150 bis 180 °C, verfahren wird.

**11.** Verfahren nach Anspruch 9 oder 10,
**dadurch gekennzeichnet**,
daß das Gemisch in den mittleren Bereich oder in die untere Hälfte der Kolonne eingespeist wird.

**12.** Verfahren zur insbesondere kontinuierlichen Herstellung von Methioninnitril, Methioninamid, Methionin oder einem seiner Salze durch Umsetzung von Methylmercaptopropionaldehyd (MMP) mit Blausäure und Ammoniak und/oder durch Umsetzung von MMP-Cyanhydrin mit Ammoniak zum Methioninnitril und gegebenenfalls Hydrolyse des Nitrils,
**dadurch gekennzeichnet,**
daß Ammoniak und Cyanhydrin bzw. Ammoniak und MMP in einem molaren Verhältnis von mindestens 4 : 1 eingesetzt werden, daß der Ammoniak in einer Konzentration über 50 Gew.-% eingesetzt wird, daß die Reaktionstemperaturen zwischen 40°C und 80°C liegen, und daß die Reaktionszeit zwischen 5 und 60 Minuten liegt.

**13.** Verfahren nach Anspruch 12,
**dadurch gekennzeichnet,**
daß die Reaktion spätestens dann beendet wird, wenn 2 % d. Th. an 2,2'-Bis-(2-methylmercaptoethyl)-iminodiacetonitril und/oder 2,2'-Bis-(2-methylmercaptoethyl)-iminodiacetonitrilmonoamid gebildet sind.

**14.** Verfahren zur Herstellung von Methioninamid, Methionin oder einem seiner Salze durch Hydrolyse von Methioninnitril zum Methioninamid in Gegenwart eines Ketons und ggf. anschließender Verseifung mit einer Base,
**dadurch gekennzeichnet,**
daß die Hydrolyse des Nitrils bezogen auf das Nitril in Gegenwart von 15 - 50 eq $H_2O$, 0,2 - 2 eq Keton, 0,1 - 1,1 eq Hydroxid und 1 - 7 eq $NH_3$ sowie bei einer Temperatur zwischen 10 und 30 °C und bei einer Reaktionszeit zwischen 10 und 90 Minuten durchgeführt wird.

15. Verfahren zur Herstellung von Methionin oder einem seiner Salze durch Umsetzung eines entsprechenden Aldehyds mit Blausäure und Ammoniak oder durch Umsetzung eines entsprechenden Aldehyds mit Blausäure und anschließender Umsetzung mit Ammoniak zum Nitril der Aminosäure, Hydrolyse des Nitrils der Aminosäure zum Amid der Aminosäure und anschließender Verseifung mit einer Base,
**dadurch gekennzeichnet**,
daß die Verseifungslösung bis 40 Minuten auf eine Temperatur über 150 °C erhitzt wird, bis der Cyanidgehalt der Lösung unter 10 ppm, bezogen auf 40 Gew.-% Aminosäurelösung, reduziert ist.

16. Verfahren nach Anspruch 15,
dadurch gekennzeichnet,
daß mind. 15 min auf ≥ 160 °C erhitzt wird.

17. Verfahren nach Anspruch 9, 10 oder 11,
**dadurch gekennzeichnet**,
daß das im Sumpf nach zumindest teilweiser Abtrennung des Ammoniaks erhaltene Sumpfprodukt ggf. unter Zusatz von frischem Keton und/oder von Wasser zur Hydrolyse des Methioninnitrils zurückgeführt wird.

## Claims

1. Process for preparing methionine or one of its salts by hydrolysing methionine nitrile to methioninamide in the presence of a ketone followed by saponification of the amide with a base, characterised in that during and/or after the saponification of the amide, ammonia, ketone and water are jointly removed at a temperature ≥ 85°C and/or by applying a vacuum.

2. Process according to claim 1, characterised in that the methionine nitrile is obtained by reacting methylmercaptopropionaldehyde (MMP) with hydrocyanic acid and ammonia or by reacting the corresponding cyanohydrin (CH) with ammonia or by reacting any suitable mixture of MMP/hydrocyanic acid and the corresponding cyanohydrin with ammonia.

3. Process according to claim 1 or 2, characterised in that the ketone is separated from the ammonia.

4. Process according to claim 3, characterised in that the substantially ketone-free ammonia is added to the nitrile synthesis according to Claim 2.

5. Process for preparing methioninamide, methionine or one of its salts by reacting methylmercaptopropionaldehyde (MMP) with hydrocyanic acid and ammonia or the corresponding cyanohydrin (CH) with ammonia or a mixture of MMP, CH, hydrocyanic acid and ammonia to form methionine nitrile, followed by hydrolysis of the nitrile in the presence of a ketone to the amide, optionally followed by saponification of the amide, separation of the ammonia and recycling of at least part thereof to the nitrile synthesis, characterised in that the ammonia is substantially freed from the ketone before being recycled.

6. Process according to claim 5, characterised in that the recycled ammonia added to the nitrile synthesis contains less than 25 meq relative to the cyanohydrin and/or aldehyde.

7. Process for preparing methionine or methioninates by hydrolysing methionine nitrile or methioninamide in the presence of a base, characterised in that the initial concentration of the amide is maintained at ≤ 25 wt.% as long as the saponification temperature is 160°C or higher.

8. Process according to claim 7, characterised in that the saponification temperature is not higher than 200°C.

9. Process according to claim 1, characterised in that in order to separate ammonia from a jointly removed ammonia/ketone/water mixture, the said mixture is fed into a column between the top and bottom thereof, the column is maintained at a pressure between 1 - 20 bar excess, the temperature at the bottom of the column is maintained between 100 and 200°C, and the ketone, water and possibly slight amounts of ammonia are withdrawn from the bottom.

10. Process according to claim 9, characterised in that it is carried out at a pressure between 5 and 18 bar excess, in

particular 9 to 15 bar excess, and at a temperature of 140 to 190°C, in particular 150 to 180°C.

11. Process according to claim 9 or 10, characterised in that the mixture is fed into the middle region or into the lower half of the column.

12. Process for in particular the continuous preparation of methionine nitrile, methioninamide, methionine or one of its salts by reacting methylmercaptopropionaldehyde (MMP) with hydrocyanic acid and ammonia and/or by reacting MMP-cyanohydrin with ammonia to form methionine nitrile and optionally hydrolysing the nitrile, characterised in that ammonia and cyanohydrin or ammonia and MMP are used in a molar ratio of at least 4 : 1, the ammonia is used in a concentration of more than 50 wt.%, the reaction temperature is between 40°C and 80°C, and the reaction time is between 5 minutes and 60 minutes.

13. Process according to claim 12, characterised in that the reaction is terminated at the latest when 2 % of theory of 2,2'-bis-(2-methylmercaptoethyl)-iminodiacetonitrile and/or 2,2'-bis-(2-methylmercaptoethyl)-iminodiacetonitrile monoamide are formed.

14. Process for preparing methioninamide, methionine or one of its salts by hydrolysing methionine nitrile to methioninamide in the presence of a ketone, optionally followed by saponification with a base, characterised in that the hydrolysis of the nitrile is carried out in the presence of, referred to the nitrile, 15 - 50 equivalents of water, 0.2 - 2 equivalents of ketone, 0.1 - 1.1 equivalents of hydroxide and 1 - 7 equivalents of ammonia, as well as at a temperature of between 10 and 30°C and at a reaction time of between 10 and 90 minutes.

15. Process for preparing methionine or one of its salts by reacting a corresponding aldehyde with hydrocyanic acid and ammonia or by reacting a corresponding aldehyde with hydrocyanic acid followed by reaction with ammonia to form the amino acid nitrile, hydrolysis of the amino acid nitrile to form the amino acid amide, and subsequent saponification with a base, characterised in that the saponification solution is heated for up to 40 minutes at a temperature above 150°C until the cyanide content of the solution is reduced to below 10 ppm referred to 40 wt. % amino acid solution.

16. Process according to claim 15, characterised in that the solution is heated for at least 15 minutes at ≥ 160°C.

17. Process according to claim 9, 10 or 11, characterised in that the bottom product obtained from the bottom of the column is recycled after at least partial separation of the ammonia, optionally with the addition of fresh ketone and/or water, to the hydrolysis of the methionine nitrile.

**Revendications**

1. Procédé pour la préparation de méthionine ou d'un de ses sels par hydrolyse du nitrile de méthionine pour obtenir l'amide de méthionine en présence d'une cétone et par saponification ultérieure de l'amide avec une base, caractérisé en ce que, pendant et/ou après la saponification de l'amide, on extrait conjointement l'ammoniac, la cétone et l'eau à une température ≥ 85°C et/ou en appliquant un vide.

2. Procédé selon la revendication 1,
caractérisé en ce qu'on obtient le nitrile de méthionine par mise en réaction de méthylmercaptopropionaldéhyde (MMP) avec de l'acide cyanhydrique et de l'ammoniac ou par mise en réaction de la cyanhydrine (CH) correspondante avec de l'ammoniac ou encore par mise en réaction d'un mélange quelconque de MMP/acide cyanhydrique et de la cyanhydrine correspondante avec de l'ammoniac.

3. Procédé selon la revendication 1 ou 2,
caractérisé en ce qu'on sépare la cétone de l'ammoniac.

4. Procédé selon la revendication 3,
caractérisé en ce qu'on achemine l'ammoniac essentiellement exempt de cétone à la synthèse du nitrile selon la revendication 2.

5. Procédé pour la préparation d'amide de méthionine, de méthionine ou d'un de ses sels par mise en réaction de méthylmercaptopropionaldéhyde (MMP) avec de l'acide cyanhydrique et de l'ammoniac ou de la cyanhydrine correspondante (CH) avec de l'ammoniac ou avec un mélange de MMP, de CH, d'acide cyanhydrique et d'ammoniac pour obtenir le nitrile de méthionine, par hydrolyse ultérieure du nitrile en présence d'une cétone pour obtenir l'amide, le cas échéant par saponification ultérieure de l'amide, séparation de l'ammoniac et recyclage de ce dernier au moins de manière partielle dans la synthèse du nitrile,
caractérisé en ce qu'on libère essentiellement l'ammoniac de la cétone avant le recyclage.

**6.** Procédé selon la revendication 5,
caractérisé en ce que l'ammoniac recyclé, renvoyé dans la synthèse du nitrile contient moins de 25 méq. par rapport à la cyanhydrine et/ou à l'aldéhyde.

**7.** Procédé pour la préparation de méthionine ou de méthioninates par hydrolyse du nitrile de méthionine, respectivement de l'amide de méthionine en présence d'une base,
caractérisé en ce qu'on maintient la concentration de départ de l'amide à une valeur $\leq$ 25% en poids pour autant que la température de saponification se situe à 160°C ou plus.

**8.** Procédé selon la revendication 7,
caractérisé en ce que la température de saponification n'est pas supérieure à 200°C.

**9.** Procédé selon la revendication 1,
caractérisé en ce que, pour la séparation de l'ammoniac à partir d'un mélange d'ammoniac-cétone-eau extrait de manière conjointe, on introduit le mélange dans une colonne entre la tête et le bas de la colonne, en ce qu'on maintient la colonne sous une pression entre 1-20 bar, en ce qu'on maintient la température de bas de colonne entre 100 et 200°C et en ce qu'on extrait l'acétone, l'eau et éventuellement des quantités minimes d'ammoniac du bas de la colonne.

**10.** Procédé selon la revendication 9,
caractérisé en ce qu'on travaille sous une pression entre 5 et 18 bar, en particulier de 9 à 15 bar, et à une température de 140 à 190°C, en particulier de 150 à 180°C.

**11.** Procédé selon la revendication 9 ou 10,
caractérisé en ce qu'on introduit le mélange dans la zone médiane et/ou dans la moitié inférieure de la colonne.

**12.** Procédé pour la préparation, en particulier en continu, du nitrile de méthionine, de l'amide de méthionine, de méthionine ou d'un de ses sels par mise en réaction de méthylmercaptopropionaldéhyde (MMP) avec de l'acide cyanhydrique et de l'ammoniac et/ou par mise en réaction de MMP-cyanhydrine avec de l'ammoniac pour obtenir le nitrile de méthionine et, le cas échéant, par hydrolyse du nitrile,
caractérisé en ce qu'on met en oeuvre l'ammoniac et la cyanhydrine, respectivement l'ammoniac et MMP dans un rapport molaire d'au moins 4:1, en ce qu'on met en oeuvre l'ammoniac en une concentration supérieure à 50% en poids, en ce que les températures réactionnelles se situent entre 40°C et 80°C, et en ce que le temps de réaction se situe entre 5 et 60 minutes.

**13.** Procédé selon la revendication 12,
caractérisé en ce que la réaction arrive à son terme au plus tard lorsqu'on obtient 2% de la théorie du 2,2'-bis-(2-méthylmercaptoéthyl)iminodiacétonitrile et/ou du monoamide du 2,2'-bis-(2-méthylmercaptoéthyl)iminodiacétonitrile.

**14.** Procédé pour la préparation d'amide de méthionine, de méthionine ou d'un de ses sels par hydrolyse du nitrile de méthionine pour obtenir l'amide de méthionine en présence d'une cétone et, le cas échéant, par saponification ultérieure avec une base,
caractérisé en ce qu'on effectue l'hydrolyse du nitrile, rapportée au nitrile, en présence de 15 à 50 éq. de $H_2O$, de 0,2-2 éq. de cétone, de 0,1-1,1 éq. d'hydroxyde et de 1-7 éq. de $NH_3$, ainsi qu'à une température entre 10 et 30°C et dans un temps de réaction entre 10 et 90 minutes.

**15.** Procédé pour la préparation de méthionine ou d'un de ses sels par mise en réaction d'un aldéhyde correspondant avec de l'acide cyanhydrique et de l'ammoniac ou par mise en réaction d'un aldéhyde correspondant avec de l'acide cyanhydrique et par mise en réaction ultérieure avec de l'ammoniac pour obtenir le nitrile de l'acide aminé, par hydrolyse du nitrile de l'acide aminé pour obtenir l'amide de l'acide aminé et par saponification ultérieure avec une base,

caractérisé en ce qu'on chauffe la solution de saponification pendant 40 minutes à une température supérieure à 150°C jusqu'à ce que l'on obtienne une réduction de la teneur de la solution en cyanure inférieure à 10 ppm, rapportée à 40% en poids de la solution d'acide aminé.

**16.** Procédé selon la revendication 15, caractérisé en ce qu'on chauffe, au moins pendant 15 minutes, à une température ≥ 160°C.

**17.** Procédé selon la revendication 9, 10 ou 11, caractérisé en ce qu'on renvoie le produit de bas de colonne obtenu au bas de la colonne après séparation au moins partielle de l'ammoniac, éventuellement avec addition de cétone fraîche et/ou d'eau pour l'hydrolyse du nitrile de méthionine.

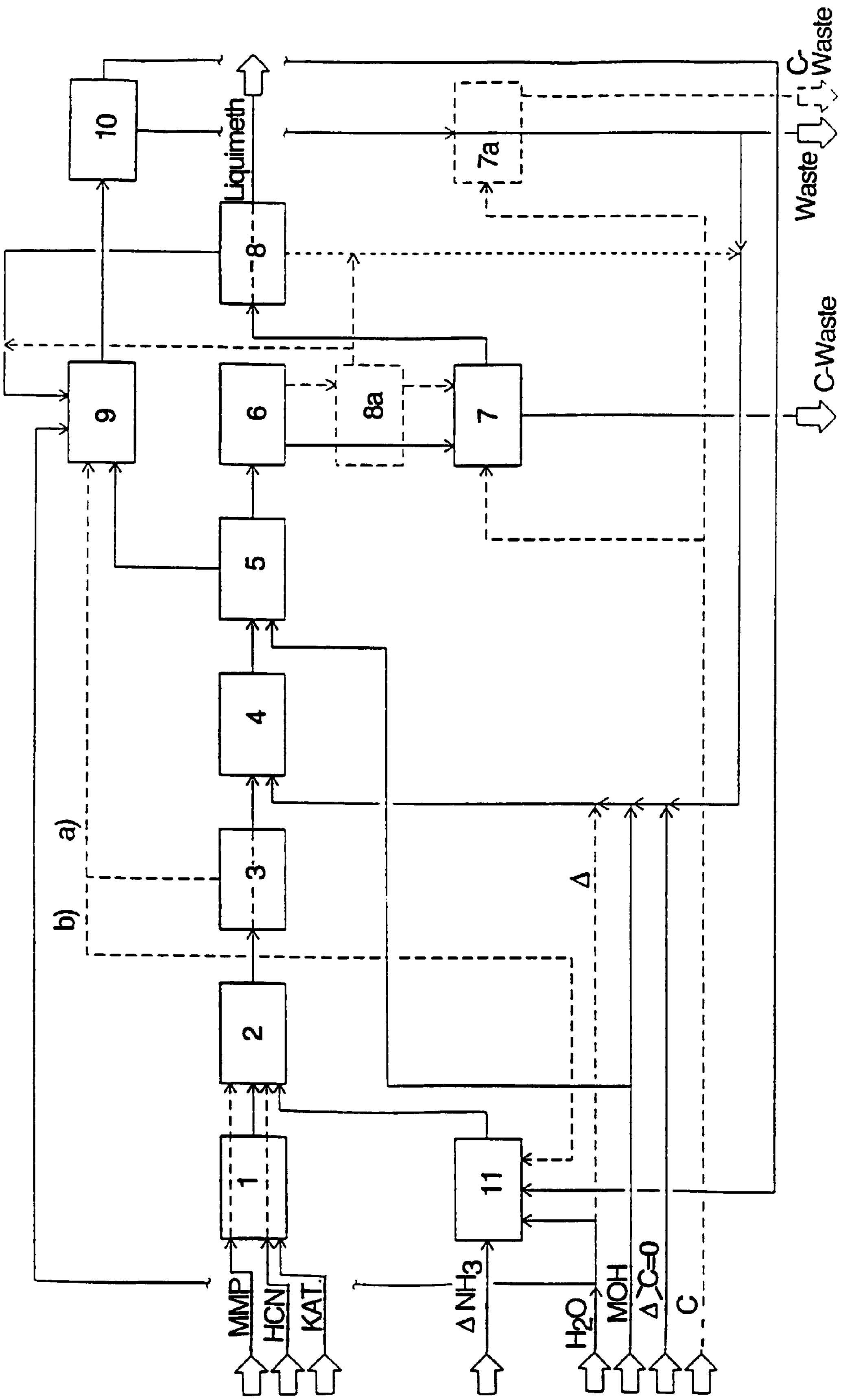
MMP
HCN
KAT.
Δ NH3
H2O
MOH
Δ C=O
C
1
2
3
4
5
6
7
7a
8
8a
9
10
11
a)
b)
Δ
Liquimeth
Waste
C-Waste
C-Waste

FIG.